# EUROPEAN PATENT APPLICATION

(11) **EP 4 470 563 A1**
(43) Date of publication of application: **04.12.2024**
(21) Application number: 23746946.5
(22) Date of filing: 24.01.2023
(51) Int. Cl.: A61K 45/00, A61K 31/136, A61K 31/198, A61K 31/223, A61P 1/00, A61P 3/00, A61P 9/00, A61P 11/00, A61P 13/00, A61P 15/00, A61P 19/00, A61P 21/00, A61P 25/00, A61P 27/00, A61P 31/00, A61P 35/00

(54) **NEW THERAPY AND PREVENTION FOR SUPPRESSING AGING-RELATED DISORDERS INCLUDING SARCOPENIA**

(30) Priority: 25.01.2022 JP 2022009538
(71) Applicant: Hayano, Motoshi, Tsukuba-shi, Ibaraki 305-0817 (JP)
(72) Inventor: TSUBOTA Kazuo, Funabashi-shi, Chiba 273-0031 (JP); HAYANO Motoshi, Tsukuba-shi, Ibaraki 305-0817 (JP)
(74) Representative: Plasseraud IP
(86) International application number: PCT/JP2023/002118
(87) International publication number: WO 2023/145735

(57) **Abstract**

Novel treatment and prevention of aging-related abnormalities, including sarcopenia, are provided. The present invention relates to pharmaceutical compositions for regulating, delaying the progression of, preventing or treating aging-related abnormalities, including sarcopenia, comprising expectorants (including compounds or pharmaceutically acceptable salts thereof or solvates thereof).

## Description

### Technical Field

This invention relates to novel treatment and prevention for the suppression of aging-related abnormalities including sarcopenia. The present invention also provides compounds, compositions, pharmaceuticals, and treatment methods that are useful for the novel treatment and prevention of sarcopenia-related diseases, disorders, symptoms, and the like.

### Background Art

Development is currently being carried out by multiple companies, mainly targeting muscle differentiation and myocyte necrosis which target factors of a member of the TGF-β family called myostatin/GDF-8. Therefore, development is underway with not only sarcopenia but also muscular dystrophy as the target diseases (patent documents 1-4, non-patent document 1).

In addition, a monoclonal antibody (bimagrumab) is under development targeting the activin receptor ACVR2B, which has completed phase II in 2017. Bimagrumab inhibits myostatin and activin A signaling and promotes muscle differentiation, thereby increasing muscle mass. However, it did not dramatically improve sarcopenia symptoms, including gait speed and grip strength, and development was discontinued in 2018.

In addition, development is underway (MK-2866) targeting androgen receptors, but the relationship between hormones and muscle is unclear, and thus the development also remains unclear.

### Prior Art Documents

### [Patent Literatures]

Patent Document 1: International Publication Pamphlet WO 2016/168613
Patent Document 2: International Publication Pamphlet WO 2013/137832
Patent Document 3: International Publication Pamphlet WO 2017/072515
Patent Document 4: International Publication Pamphlet WO 2015/162590

### [Non-patent Literatures]

[Non-Patent Document 1] Arounleut P, Bialek P, Liang LF, Upadhyay S, Fulzele S, Johnson M, Elsalanty M, Isales CM, Hamrick MW. A myostatin inhibitor (propeptide-Fc) increases muscle mass and muscle fiber size in aged mice but does not increase bone density or bone strength. Exp Gerontol. 2013 Sep;48(9):898-904.

### [Summary of the Invention]

### [Solution to the Problem]

In proceeding with research relating to novel treatments and prevention of sarcopenia-related diseases, disorders, symptoms, and the like, the present inventor has conducted intensive research and numerous experiments over many years on various compounds, their pharmaceutically acceptable salts, and their solvates, without being limited by molecular mechanisms and mechanisms of action that have been conventionally studied. In particular, when intensive experiments were conducted on compounds used as expectorants, the present inventor found that the compounds had the unexpected effect of increasing muscle strength and cognitive ability, and confirmed the suitability thereof for the purpose of treating sarcopenia, and also found that expectorant compounds improve frailty, including cognitive function, bone density, gray hair and dermatitis, which are aging-related symptoms, thereby completing the present invention.

### (Item 1)

Thus, the present invention provides compositions for the purpose of regulation, delay of progression, prevention, or treatment of sarcopenia-related disease, disorder, or symptom (or broadly, aging-related abnormalities including sarcopenia, the same hereinafter), comprising a so-called expectorant (including compounds, pharmaceutically acceptable salts, or solvates thereof.). Compounds constituting such an expectorant include the compounds themselves, pharmaceutically acceptable salts, or solvates thereof. If the expectorant of the present invention includes an acidic group or a basic group, the expectorant can be reacted with a base or an acid and converted into a basic salt or an acidic salt; thus, the term "pharmaceutically acceptable salts" refers to such salts.

In the "pharmaceutically acceptable salts," examples of "basic salts" include: alkali metal salts such as sodium salt, potassium salt, and lithium salt; alkaline earth metal salts such as magnesium salt and calcium salt; organic base salts such as N-methylmorpholine salt, triethylamine salt, tributylamine salt, diisopropylethylamine salt, dicyclohexylamine salt, N-methylpiperidine salt, pyridine salt, 4-pyrrolidinopyridine salt, and picoline salt; and aminoacid salts such as glycinate, lysinate, argininate, omithinate, glutamate, and aspartate.

In addition, examples of "acid salts" include: hydrogen halide salts such as hydrofluoride, hydrochloride, hydrobromide, and hydroiodide; inorganic acid salts such as nitrate, perchlorate, sulfate, and phosphate; lower alkane sulfonates such as methanesulfonate, trifluoromethanesulfonate, and ethanesulfonate; aryl sulfonates such as benzenesulfonate and p-toluenesulfonate; organic acid salts such as acetate, malate, fumarate, succinate, citrate, ascorbate, tartrate, oxalate, and maleate; and amino acid salts such as glycinate, lysinate, argininate, ornithinate, glutamate, and aspartate.

### (Item 2)

In addition, the expectorant may be, for example, one or more compounds selected from the group consisting of L-ethylcysteine, ambroxol, L-carbocisteine, and pharmaceutically acceptable salts thereof.

### (Item 3)

Of the foregoing, the compound is preferably one or more compounds selected from the group consisting of L-ethylcysteine hydrochloride represented by the following general formula (1) and/or ambroxol hydrochloride represented by the following general formula (2), and pharmaceutically acceptable salts thereof.

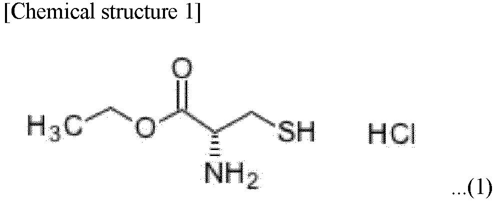

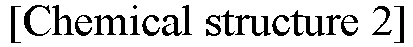

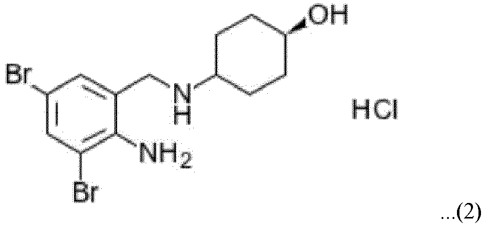

### (Item 4)

The composition according to any one of the preceding claims, wherein the aging-related abnormalities including sarcopenia includes at least one selected from the group consisting of a disease, disorder, or symptom caused by impaired muscle strength; a disease, disorder, or symptom caused by impaired energy metabolism; a disease, disorder, or symptom caused by impaired nervous system; and a disease, disorder, or symptom caused by an impaired secretory organ.

### (Item 5)

The composition according to any one of the preceding claims, wherein the aging-related abnormalities including sarcopenia includes at least one selected from the group consisting of primary sarcopenia, secondary sarcopenia, and a disease, disorder, or symptom associated therewith.

### (Item 6)

The composition according to any one of the preceding claims, wherein the aging-related abnormalities including sarcopenia is selected from the group consisting of related lifestyle diseases (non-wasting diseases), wasting diseases, tumor-related diseases (including cancer cachexia), infectious diseases and related diseases such as COVID-19, locomotor diseases, neurodegenerative diseases, cognitive impairments, and aging-related impairments (including malnutrition, frailty, spinal and lung injury, inactivity, reduced activity, disuse syndrome, and invasion such as trauma and surgery).

### (Item 7)

The composition according to any one of the preceding claims, wherein the aging-related abnormalities including sarcopenia is that of a system selected from the group consisting of: digestive system (such as digestive tract), circulatory system, respiratory system (such as vocal organs), urinary system, reproductive system, endocrine system, sensory system, cerebral nervous system, and motor system (such as bones, joints, ligaments, and muscles).

### (Item 8)

The composition according to any one of the preceding claims, wherein the aging-related abnormality including sarcopenia is of a system selected from the group consisting of hair loss and gray hair.

### (Item 9)

The composition according to any one of the preceding claims, wherein the compounds, pharmaceutically acceptable salts, or solvates thereof can be orally administered.

### (Item 10)

The composition according to any one of the preceding claims, wherein the regulation, delay of progression, prevention, or treatment includes regulation, delay of progression, prevention, or treatment of at least one of muscle mass loss, decreased (muscle) endurance, reduced short-term memory, bone density loss, and osteoporosis.

### (Item 11)

The composition according to any one of the preceding claims, wherein the regulation, delay of progression, prevention, or treatment includes regulation, delay of progression, prevention, or treatment of at least one of muscle mass loss and/or decreased (muscle) endurance.

In the present invention, one or more features listed in the claims above are provided in any combination thereof, in addition to the explicitly stated combinations, all of which are intended as falling within the technical scope of the present invention. A person skilled in the art would be able to easily understand further embodiments and advantages of the present invention on the basis of the following detailed description, as necessary. For example, the compositions according to the present invention may be administered in combination with at least one of exercise therapy, diet therapy, and other drug therapy, or may be administered in combination with exercise therapy.

### [Effects of the Invention]

The present invention achieves, for the first time, a technology for the treatment or prevention of sarcopenia and its related diseases, disorders, and symptoms. The present invention also achieves, for the first time, a technology for the treatment or prevention of age-related diseases, disorders, and symptoms. In other words, this invention realizes a new treatment or prevention technology for the suppression of aging-related abnormalities, including sarcopenia.

More specifically, treatment and prophylaxis according to the present invention includes improvement of cognitive function, and is applicable to all dementias, including Alzheimer's disease. For example, osteoporosis, including primary and secondary osteoporosis; dermatitis, including pruritus, contact dermatitis, seborrheic dermatitis, sebaceous deficiency dermatitis, eczema nummularis, lichen simplex chronicus, erythroderma, psoriasis, and hives; and hearing abnormalities, including age-related hearing loss; gray hair; and hair loss, etc. can also be treated or prevented.

### [Brief Description of Drawings]

[Figure 1] This figure is a diagram showing improvement of sarcopenia by means of administration of L-ethylcysteine hydrochloride, in which endurance (muscle strength) of aged mice (79 weeks) was measured using a treadmill. Cases in which phosphate buffered saline (PBS) was administered have also been measured as a control.
[Figure 2] This figure is a diagram showing gene regulation of muscle in mice administered with L-ethylcysteine hydrochloride, showing (158) genes with increased expression and (92) genes with decreased expression by means of administration of L-ethylcysteine hydrochloride (FDR < 0.05).
[Figure 3] This figure is a diagram showing gene regulation of muscle in mice administered with L-ethylcysteine hydrochloride, showing a pathway analysis diagram of genes with increased expression by means of administration of L-ethylcysteine hydrochloride.
[Figure 4] This figure is a diagram showing gene regulation of muscle in mice administered with L-ethylcysteine hydrochloride, showing a pathway analysis diagram of genes with decreased expression by means of administration of L-ethylcysteine hydrochloride.
[Figure 5] This figure is a diagram showing gene regulation of muscle in mice administered with L-ethylcysteine hydrochloride, showing an analysis of upstream transcription factors of genes with altered expression.
[Figure 6] This figure is a diagram showing a list of related diseases that are registered in MedlinePlus and that are correlated with differentially expressed genes due to L-ethylcysteine hydrochloride.
[Figure 7] This figure is a diagram showing improvement of sarcopenia by means of administration of ambroxol hydrochloride, in which endurance (muscle strength) of aged mice (77 weeks) was measured using a treadmill. Cases in which phosphate buffered saline (PBS) was administered have also been measured as a control.
[Figure 8] This figure is a diagram showing improvement of sarcopenia by means of administration of ambroxol hydrochloride, in which cognitive ability of aged mice (77 weeks) was measured using a contextual fear conditional test (CFC test). Cases in which phosphate buffered saline (PBS) was administered have also been measured as a control.
[Figure 9] This figure is a diagram showing gene regulation of muscle in mice administered with ambroxol hydrochloride, showing (164) genes with increased expression and (204) genes with decreased expression by means of administration of ambroxol hydrochloride (FDR < 0.05).
[Figure 10] This figure is a diagram showing the top 50 genes with altered gene expression in muscle of mice administered with ambroxol hydrochloride.
[Figure 11] This figure is a diagram showing a pathway analysis diagram of genes with increased gene expression in muscle of mice administered with ambroxol hydrochloride.
[Figure 12] This figure is a diagram showing gene regulation of muscle in mice administered with ambroxol hydrochloride, showing an analysis of upstream transcription factors of genes with altered expression.
[Figure 13] This figure is a diagram showing gene regulation of myoblastic cells in mice administered with ambroxol hydrochloride, showing (53) genes with increased expression and (54) genes with decreased expression by means of administration of ambroxol hydrochloride (Q < 0.05).
[Figure 14] This figure is a diagram showing the top 30 genes with altered gene expression in myoblastic cells of mice administered with ambroxol hydrochloride.
[Figure 15] This figure is a diagram showing a pathway analysis diagram of genes with increased gene expression in myoblastic cells of mice administered with ambroxol hydrochloride.
[Figure 16] This figure is a diagram showing a pathway analysis diagram of genes with decreased gene expression in myoblastic cells of mice administered with ambroxol hydrochloride.
[Figure 17] This figure is a diagram showing gene regulation of myoblastic cells in mice administered with ambroxol hydrochloride, showing an analysis of upstream transcription factors of genes with altered expression.
[Figure 18] This figure shows the result of a frailty index test (state of skin (hair, skin)) of mice administered with ambroxol hydrochloride.
[Figure 19] This figure shows the result of a frailty index test (muscle strength (grip force)) of mice administered with ambroxol hydrochloride.
[Figure 20] This figure is a diagram showing the result of a frailty index test (osteoporosis (physical / musculoskeletal)) of mice administered with ambroxol hydrochloride.
[Figure 21] This figure is a diagram showing the result of a frailty index test (hearing loss) of mice administered with ambroxol hydrochloride.
[Figure 22] This figure shows the evaluation results of the frailty index test (alopecia) in mice administered with L-carbocysteine.
[Figure 23] This figure shows the evaluation results of the frailty index test (improvement of gray hair) in mice administered with L-carbocysteine.
[Figure 24] This figure shows the evaluation results of the frailty index test (improvement of hair loss) in mice treated with L-carbocysteine.
[Figure 25] This figure shows the evaluation results of the frailty index test (Improvement of bones and muscles) in L-carbocysteine-treated mice.
[Figure 26] This figure shows the evaluation results of the frailty index test (improvement of frailty) in mice administered with L-carbocysteine.

### Detailed Description of the Invention

The present invention will be described below with reference to the preferred embodiments. Throughout the present Specification, it should be understood that expressions in the singular form include the concept of their plural forms, unless otherwise specified. Accordingly, singular articles (for example, "a," "an," and "the" in the case of English) should be understood as also including the concept of the plural forms thereof, unless otherwise specified. In addition, it should be understood that terms used in the present Specification are used in the sense commonly used in the relevant field, unless otherwise specified. Accordingly, unless otherwise defined, all technical and scientific terms used in the present Specification have the same meanings as commonly understood by a person skilled in the art of the field to which the present invention belongs. In there is a contradiction, the present Specification (including the definitions) takes precedence.

Definitions of terms and/or basic technical contents specifically used in the present Specification will be described below as appropriate.

### (Definition of terms)

In the present Specification, "sarcopenia" refers to a disease that is diagnosed when there is, in addition to 1. evidence of low muscle mass, there is 2. low muscle strength or 3. low physical function (European Working Group on Sarcopenia in Older People (EWGSOP)). Since skeletons of Westerners and Asians are different, Asian-specific diagnostic criteria that can be applied to Japanese physiques have been created (Sports Medicine 27(9), 2-11, 2015-11, Bookhouse HD), by the Asian Working Group for Sarcopenia (AWGS) in 2014. Sarcopenia is classified into "primary sarcopenia" caused by aging, and "secondary sarcopenia," having causes other than aging. In addition to aging, it can also be caused by activities of daily living, disease, and nutritional state, such as disuse caused by being bedridden or reduced activity, diseases such as cancer, ischemic heart failure, end-stage renal failure, and endocrine disorders, malabsorption of nutrients, loss of appetite due to gastrointestinal disorders or side effects of medication, insufficient intake of energy and protein, and other conditions in which activity decreases or is lost due to neurodegeneration (such as ALS). This refers to "loss of muscle strength" of the whole body, such as grip force, lower limb muscles, and core muscles, as a result of decrease in muscle mass caused by aging or disease. As a result, physical function declines, such as slower walking speed and needing canes or handrails.

In addition to the function of "muscle strength," muscles also have elements of "energy metabolism" and as a "secretory organ." If muscle mass or muscle function decreases, functions of internal organs, such as the heart and the respiratory system, decreases from the perspective of "muscle strength," and bones in the joints are put under strain, leading to arthritis. From the perspective of "energy metabolism," decrease in muscle results in the inability to use the fat and sugar in the blood, leading to obesity. Inability to metabolize fat leads to metabolic diseases such as myocardial infarction. NAMPT then synthesizes NAD from NMN in muscles. If NAD decreases, brain function, metabolic function, etc. will also decrease. From the perspective of a "secretory organ," muscles secrete inflammatory substances such as IL-6, and growth factors such as insulin-like growth factor 1 (IGF-1) and BDNF, thereby affecting cancer, bones, nerves, and blood cell proliferation. (Refer to Hoffmann C, Weigert C. Skeletal Muscle as an Endocrine Organ: The Role of Myokines in Exercise Adaptations. Cold Spring Harb Perspect Med. 2017;7(11):a029793. Published 2017 Nov 1. doi:10.1101/cshperspect.a029793). The importance of treating sarcopenia is not only about restoring muscle strength, but also about the possibility of treatment from the perspective of recovery of muscle as a metabolic and secretory organ, as well as the perspective of systemic aging. It can be said that because of that, sarcopenia patients suffer a wide range of disease development.

Muscle mass is maintained by means of repeated muscle proteosynthesis and proteolysis. Muscle mass also decreases when there is a decrease in factors necessary for muscle proteosynthesis or when muscle proteolysis exceeds muscle proteosynthesis. Sarcopenia develops when there is, as a result of aging, a reduction in sex hormones related to muscle growth, death of cells (apoptosis) necessary for muscle function, or occurrence of mitochondrial dysfunction that is combined with factors of muscle atrophy (cachexia) caused by disuse, malnutrition, or a wasting disease such as cancer and diabetes. Sarcopenia can also be caused by loss of motor nerves that function to transmit instructions from the brain to the muscles and by the effects of hormones related to muscle growth, such as corticosteroids, growth hormones (GH), insulin-like growth factor 1 (IGF-1), thyroid dysfunction, and insulin resistance. It is thought that various diseases causing an increase in inflammatory cytokines and accelerating muscle proteolysis can also lead to the development of sarcopenia.

In the present Specification, "sarcopenia-related disease, disorder, or symptom" refers to a disease, disorder, or symptom associated with sarcopenia or to one or more of the following characteristics of sarcopenia: muscle strength impairment, energy metabolism impairment, and secretory organ impairment. Sarcopenia-related diseases, disorders, or symptoms can be discussed separately in terms of diseases, disorders, or symptoms caused by muscle strength impairment; diseases, disorders, or symptoms caused by energy metabolism impairment; and diseases, disorders, or symptoms caused by secretory organ impairment (however, there are diseases, disorders, or symptoms that pertain to two or more categories.), which are to be considered as comprehensive expressions. In addition, sarcopenia-related diseases, disorders, or symptoms can also be discussed in terms of primary sarcopenia, secondary sarcopenia, and associated diseases, disorders, or symptoms thereof. The present invention is broadly aimed at sarcopenia patients, and examples of sarcopenia-related diseases, disorders, or symptoms include, but are not limited to: sarcopenia-related lifestyle diseases (non-wasting diseases), wasting diseases, tumor-related diseases (including cancer cachexia), infectious diseases and related diseases such as COVID-19, locomotor diseases, neurodegenerative diseases (including ALS), cognitive impairments, and other related impairments (defined to include malnutrition, frailty, spinal and lung injury, inactivity, reduced activity, disuse syndrome, and invasion such as trauma and surgery).

In the present Specification, "aging-related disease, disorder, or symptom" and "age-related disease, disorder, or symptom" are used interchangeably and refer to diseases, disorders, or symptoms that develop in association with aging. Examples of aging-related diseases, disorders, or symptoms include sarcopenia, lifestyle diseases (non-wasting diseases), wasting diseases, locomotor diseases, neurodegenerative diseases, cognitive impairment, and other related disorders, more specifically, sarcopenia, dementia (including reduced short-term memory.), metabolic diseases, rheumatoid arthritis, osteoporosis, muscle mass loss, hair loss, and the like.

In the present Specification, "disease, disorder, or symptom caused by muscle strength impairment" refers to any disease, disorder, or symptom related to a decrease in muscle mass or quality (muscle function). For example, it includes, from the perspective of "muscle strength," decreases in the function of internal organs such as the heart and respiratory system, as well as diseases, disorders, or symptoms such as arthritis caused by strain imparted on bones in joints. Examples of diseases, disorders, or symptoms caused by muscle strength impairment include neurogenic muscular atrophy, myogenic muscular atrophy (such as muscular dystrophy, congenital myopathy, mitochondrial encephalomyopathy, myopathy due to congenital metabolic abnormality, and other myopathies), and the like.

In the present Specification, "disease, disorder, or symptom caused by energy metabolism impairment" refers to any disease, disorder, or symptom caused by the impairment of energy metabolism. A decrease in muscle leads to an inability to use the fat and sugar in the blood, resulting in obesity, and an inability to metabolize fat leads to metabolic diseases such as myocardial infarction, but NAMPT synthesizes NAD from NMN in muscle. Since brain function and metabolic function decrease if NAD decreases, any disease caused by such functions can be exemplified as a disease, disorder, or symptom caused by energy metabolism impairment related to sarcopenia. Examples of diseases, disorders, or symptoms caused by energy metabolism impairment include diabetes, dyslipidemia, obesity, metabolic syndrome, osteoporosis, fatty liver, hyperuricemia, and hypertension.

In the present Specification, "disease, disorder, or symptom caused by secretory organ impairment" refers to any disease, disorder, or symptom related to an impairment of muscle serving as a secretory organ that secretes or produces substances from the muscles. Muscles secrete inflammatory substances such as IL-6, and growth factors such as insulin-like growth factor 1 (IGF-1) and BDNF, so there is an effect on cancer, bones, nerves, and blood cell proliferation (Hoffmann C, Weigert C. Skeletal Muscle as an Endocrine Organ: The Role of Myokines in Exercise Adaptations. Cold Spring Harb Perspect Med. 2017;7(11):a029793. Published 2017 Nov 1. doi:10.1101/cshperspect.a029793 = https://www.ncbi.nlm.nih.gov/pmc/articles/PMC5666622/). Impairment of these functions as a secretory organ causes diseases, disorders, or symptoms. Diseases, disorders, or symptoms associated with the foregoing are included in the diseases, disorders, or symptoms caused by secretory organ impairment. Examples of diseases, disorders, or symptoms caused by secretory organ impairment include diabetes, pancreatitis, ulcerative colitis, diarrhea, and the like.

In the present Specification, "disease, disorder, or symptom caused by nervous system impairment" refers to any disease, disorder, or symptom related to an impairment of nerves related to muscles. Muscles contract in response to signals from the nervous system, and muscles are closely related to nerves. Impairment of these functions as a nervous system causes diseases, disorders, or symptoms. Diseases, disorders, or symptoms associated with the foregoing are included in the diseases, disorders, or symptoms caused by secretory organ impairment. Examples of diseases, disorders, or symptoms caused by nervous system impairment include neurodegenerative diseases (including ALS), cognitive impairment, and the like.

In the present Specification, "primary sarcopenia" refers to sarcopenia that has no apparent cause other than aging.

In the present Specification, "secondary sarcopenia" refers to sarcopenia that has a cause other than aging, examples of which include activity-related sarcopenia (which could be caused by being bedridden, inactive lifestyle, or weightlessness), disease-related sarcopenia (which may accompany severe organ failure (heart, lungs, liver, kidneys, brain), inflammatory diseases, malignant tumors, or endocrine diseases), and nutrition-related sarcopenia (caused by insufficient energy and/or protein intake due to malabsorption, gastrointestinal diseases, and the use of medications that cause loss of appetite, and the use of medications that cause loss of appetite).

In the present Specification, compounds refer to compounds represented by the above formula (1), compounds represented by the above formula (2), and compounds, pharmaceutically acceptable salts, or solvates thereof, which are separately defined in the present Specification.

In the present Specification, "exercise therapy" refers to therapy through exercise. Non-pharmaceutical interventions for sarcopenia include exercise and diet therapy involving Vitamin D, Omega-3, and the like.

In the present Specification, "diet therapy" refers to therapy based on diet. Non-pharmaceutical interventions for sarcopenia include exercise and diet therapy involving Vitamin D, Omega-3, and the like.

In the present Specification, "another drug therapy" refers to therapy with a drug other than the "compounds represented by the above formula (1) and compounds represented by the above formula (2)" of the present invention. Using a plurality of the compounds represented by the above formula (1), and compounds represented by the above formula (2) is not referred to as another drug therapy, but rather corresponds to a case of using a plurality of the compounds, etc., of the present invention.

In the present Specification, "prevention" is the act of administering the active ingredients of the present invention to a healthy person who has not developed a disease with the aim of preventing the onset of the disease, for example.

In the present Specification, "treatment" is the act of administering the active ingredients of the present invention to a person (patient) who has been diagnosed by a doctor as having developed a disease.

In the present Specification, "regulate" means that the state of the disease, etc. (for example, a sarcopenia-related disease, disorder, or symptom) of a subject who has developed the disease changes, preferably improves, when the compound of the present invention is administered compared to when the compound is not administered.

In the present Specification, "suppression of progression" means that the severity of the disease, etc. (for example, a sarcopenia-related disease, disorder, or symptom) of a subject who has developed the disease does not worsen, and preferably improves.

In the present Specification, "kit" refers to a unit in which parts to be provided (for example, compound, pharmaceutical product, composition, etc., and instruction manual) are provided, usually separated into two or more compartments. This kit form is preferable when the aim is to provide compositions that should not be provided in a mixed state, for reasons of stability, etc., but rather should be admixed immediately before use. Alternatively, these kits are also preferable when being provided in combination with an agent used to identify the patient beforehand, as a companion drug. It is advantageous if such kits preferably include instructions or instruction manuals describing how to use the provided parts, or, how to dispose of the reagents or waste liquids after use. In the present Specification, when a kit is used as a pharmaceutical kit, the kit usually includes instructions, etc., describing the administration method of the pharmaceutical product, and the like.

In the present Specification, "instructions" describe how to use the present invention to the user. These instructions include language instructing the method of use (for example, administration) of the compounds, etc., of the present invention. If necessary, these instructions are created in accordance with a format specified by the regulatory agency of the country in which the present invention is implemented (such as the Ministry of Health, Labor and Welfare or the Ministry of Agriculture, Forestry and Fisheries in Japan, and the Food and Drug Administration (FDA) or the Department of Agriculture (USDA) in the United States), and it will be clearly stated that the instructions have been approved by said regulatory agency. The instructions can be provided in paper form, but no limitation is imposed thereby; for example, the instructions may be provided in the form of electronic media (such as an Internet web page, E-mail., etc.).

Here, the pharmaceutical composition according to the present invention comprises an expectorant, pharmaceutically acceptable salts, or solvates thereof, and the expectorant comprises one or more compounds selected from the group consisting of ethylcysteine, ambroxol, carbocisteine, and pharmaceutically accepted salts thereof. Said compounds include a compound represented by the following general formula (1) and/or a compound represented by the following general formula (2).

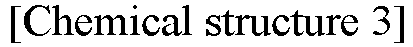

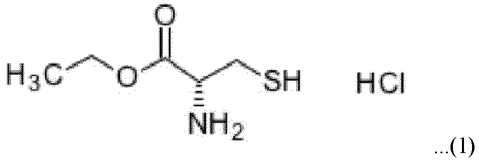

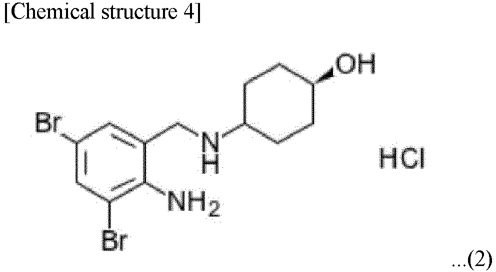

The compound presented by formula (1) has the common name L-ethylcysteine hydrochloride (Ethyl L-Cysteine Hydrochloride), and the molecular formula thereof is, for example, C5H11NO2S•HCL. For example, "CYSTANIN" manufactured by Nipro ES Pharma Co., Ltd. can be suitably used as said compound. In addition, the compound represented by formula (2) has the common name ambroxol hydrochloride, and the molecular formula thereof is, for example, C13H18Br2N2O•HCl.

The compounds of the present invention can be prepared by conventional preparation methods, including, but not limited to, those described below. These manufacturing methods can be improved as appropriate, on the basis of knowledge of a person skilled in synthetic organic chemistry. In the manufacturing methods described below, a compound used as the raw material may be substituted by a salt thereof, so long as there is no interference with the reaction. The present invention also relates to such manufacturing methods.

In the manufacturing method of the present invention, even if the use of a protective group is not specifically specified, if a functional group other than the reactive site would change under the reaction conditions, or, if it is unsuitable for carrying out post-reaction treatments, functional groups other than the reactive site may be protected as necessary, and then deprotected after the completion of the reaction or after a series of reactions has been carried out, to obtain the target compound. Conventional protective groups described in the document (T. W. Greene and P. G. M. Wuts, "Protective Group in Organic Synthesis", 3rd Ed., John Wiley and Sons, Inc., New York (1999)), and the like, may be used as the protective groups used in these processes. Introduction and removal of protective groups can be performed by means of methods commonly used in synthetic organic chemistry (such as the methods described in the document mentioned above) or analogous methods thereof.

The starting materials and intermediate materials in the manufacturing method of the present invention can be commercially purchased, or, be obtained by means of methods disclosed in known literature or by means of synthesis from known compounds following known methods. In addition, these starting materials and intermediate materials may be substituted by the salts thereof, so long as there is no interference with the reaction.

The intermediate materials and the target compound in the manufacturing method of the present invention can be converted to other compounds included in the present invention by appropriately converting functional groups thereof. The conversion of functional groups in this case can be performed by means of methods commonly used in synthetic organic chemistry (such as the methods disclosed in R. C. Larock, "Comprehensive Organic Transformations", 2nd Ed., John Wiley and Sons, Inc., New York (1999)) or analogous methods thereof.

Of the raw materials and intermediate materials in each manufacturing method described above, those for which the manufacturing method is not particularly described are commercially available compounds, or can be synthesized from commercially available compounds following methods known to a person skilled in the art, or analogous methods thereof.

The preferred embodiments of the present invention are provided to facilitate better understanding of the present invention, but the scope of the invention should not be limited to the following description. Accordingly, it will be apparent that those skilled in the art can make appropriate modifications within the scope of the present invention based on the descriptions in the present Specification. In addition, the following embodiments of the present invention can each be used independently or in combination.

In one aspect, the present invention provides compositions, pharmaceuticals, methods, compounds, uses, and the like, for the regulation, delay of progression, prevention, or treatment of a sarcopenia-related disease, disorder, or symptom, and comprises or uses a compound represented by the above formula (1) and/or a compound represented by the above formula (2). The present invention has been completed and is provided based on the unexpected discovery that compounds represented by the above formula (1) and compounds represented by the above formula (2) can regulate, delay the progress of, prevent, or treat sarcopenia-related diseases, disorders, or symptoms.

The compounds used in the present invention are compounds represented by the above formula (1), compounds represented by the above formula (2), and compounds, pharmaceutically acceptable salts, or solvates (including, for example, hydrates) thereof. Therefore, in a preferred embodiment, compounds of the present invention are compounds represented by the above formula (1) and/or compounds represented by the above formula (2).

In one embodiment, the sarcopenia-related disease, disorder, or symptom that could be regulated, delayed in its progression, prevented, or treated in the present invention includes at least one selected from the group consisting of a disease, disorder, or symptom caused by impaired muscle strength; a disease, disorder, or symptom caused by impaired energy metabolism; and a disease, disorder, or symptom caused by an impaired secretory organ.

In specific embodiments, examples of sarcopenia-related diseases, disorders, or symptoms include: sarcopenia-related lifestyle diseases (non-wasting diseases), wasting diseases, tumor-related diseases (including cancer cachexia), infectious diseases and related diseases such as COVID-19, locomotor diseases, neurodegenerative diseases (including ALS), cognitive impairments, and other related impairments (defined to include malnutrition, frailty, spinal and lung injury, inactivity, reduced activity, disuse syndrome, and invasion such as trauma and surgery). These include, but are not limited to, respiratory diseases (such as respiratory failure), visceral (or urinary system) diseases (such as chronic kidney disease (CKD)), bone-related diseases (osteoporosis, etc.), and muscle-related diseases (such as dystrophy, myofibrillar myopathy, and ALS).

Examples of diseases, disorders, or symptoms that are the subject of the present invention include, but are not limited to, diseases, disorders, or symptoms related to the digestive system (gastrointestinal tract), circulatory system, respiratory system (including the vocal organs), urinary system, reproductive system, endocrine system, sensory system, nervous system, motor system (bones, joints, ligaments, muscles, etc.), and neoplastic (cancer, tumors, etc.) system, which may be directly or indirectly related to sarcopenia through, for example, muscle strength, energy metabolism, or the secretory system.

In another aspect, the present invention provides compositions, methods, compounds, or uses for the regulation, delay of progression, prevention, or treatment of an aging-related disease, disorder, or symptom, comprising a compound represented by the above formula (1) and/or a compound represented by the above formula (2).

In one embodiment, the aging-related disease, disorder, or symptom is selected from the group consisting of sarcopenia, lifestyle diseases (non-wasting diseases), wasting diseases, tumor-related diseases (including cancer cachexia), infectious diseases and related diseases such as COVID-19, locomotor diseases, neurodegenerative diseases, cognitive impairments, and other related impairments (same as above). In yet another embodiment, the aging-related disease, disorder, or symptom is selected from sarcopenia, dementia, metabolic diseases, rheumatoid arthritis, osteoporosis, muscle mass loss, and hair loss.

In one embodiment, the compounds used in the present invention are compounds represented by the above formula (1) and/or compounds represented by the above formula (2).

In other embodiments, the compounds represented by the above formula (1) and/or compounds represented by the above formula (2) used in the present invention can be orally administered.

In some of the embodiments, the regulation, delay of progression, prevention, or treatment may be confirmed by regulation of at least one gene in the subject.

In one aspect, the present invention provides compositions for the regulation, delay of progression, prevention, or treatment of an aging-related disease, disorder, or symptom, comprising a compound represented by the above formula (1) and/or a compound represented by the above formula (2). In one embodiment, the aging-related disease, disorder, or symptom is selected from: sarcopenia, lifestyle diseases (non-wasting diseases), wasting diseases, tumor-related diseases (including cancer cachexia), infectious diseases and related diseases such as COVID-19, locomotor diseases, neurodegenerative diseases, cognitive impairments, and other related impairments (any impairment defined as including malnutrition, frailty, spinal and lung injury, inactivity, reduced activity, disuse syndrome, and invasion such as trauma and surgery). In specific embodiments, the aging-related disease, disorder, or symptom is selected from sarcopenia, dementia, metabolic diseases, rheumatoid arthritis, osteoporosis, muscle mass loss, and hair loss.

In one aspect, the present invention provides compositions to be used for diagnosing or testing a subject for sarcopenia-related diseases, disorders, or symptoms, wherein the compositions include means or reagents for identifying the presence/absence or the level of at least one gene expression, selected from the group consisting of a therapeutic gene cluster.

In one embodiment of the present invention, the therapeutic gene cluster can be classified into various clusters, as conventionally known.

In a preferred embodiment, keratinization-related gene cluster and extracellular structure organization-related gene cluster can be used as the therapeutic gene cluster.

In one aspect, the present invention provides compositions for the regulation, delay of progression, prevention, or treatment of a sarcopenia-related disease, disorder, or symptom, comprising a substance or factor that regulates at least one gene.

In another aspect, the present invention is a method for screening a substance or factor used for regulation, delay of progression, prevention, or treatment of a sarcopenia-related disease, disorder, or symptom, said method comprising:
A) a step for administrating a candidate substance or factor to a subject,
B) a step for confirming regulation of at least one gene in the subject, and
C) a step for identifying, on the basis of the result of B), that the candidate substance or factor is a substance or factor that could be capable of regulating, delaying the progress, preventing, or treating a sarcopenia-related disease, disorder, or symptom.

The compounds represented by the above formula (1), compounds represented by the above formula (2), pharmaceuticals, and compositions of the present invention can be administered in any dosage form. Preferably, the dosage form is oral administration, but no limitation is imposed thereby.

The "targets" or "subjects" to whom administration is conceivable include, but are not limited to, humans (that is, males or females of any age group, for example pediatric subjects (such as infants, children, adolescents) or adult subjects (such as young adults, middle-aged adults, or elderly adults)) and/or other non-human animals, for example mammals (such as primates (such as rhesus monkeys, macaques), commercially relevant mammals, such as cattle, pig, horse, sheep, goat, cat, and/or dog) and birds (such as commercially relevant birds, such as chicken, duck, geese, and/or turkey), reptiles, amphibians, and fish. In some embodiments, a non-human animal is a mammal. The non-human animal could be male or female at any stage of development. The non-human animal can be a transgenic animal.

When used in the present specification, unless otherwise specified, the terms "treat," "treating," and "treatment" refer to acts carried out while the subject is afflicted with a disease, disorder, or symptom. In the present Specification, an act of reducing the severity of a disease, disorder, or symptom, or delaying or slowing the progression of a disorder, is called "delaying the progress," and an act that occurs before the subject begins to suffer from a disease, disorder, or symptom, and that inhibits the disease, disorder, or symptom or reduces the severity of a disorder, is called "prevention."

In general, the "effective amount" of a compound is the amount that is sufficient to cause a desired biological response, that is, to treat a disorder. As is obvious to a person skilled in the art, the effective amount of a compound of the present invention could change depending on factors such as the desired biological endpoint, the pharmacokinetics of the compound, the disorder to be treated, the mode of administration, and the age and health as well as the subject. The effective amount includes that for treatment as well as prophylactic treatment.

When used in the present specification, unless otherwise specified, the "therapeutically effective amount" of a compound refers to an amount that is sufficient to provide a therapeutic effect in treating a disorder, or to delay or minimize one or more symptoms associated with the disorder. The therapeutically effective amount of a compound means the amount of a therapeutic agent that is able, either alone or in combination with another therapy, to provide a therapeutic effect in the treatment of a disorder. The term "therapeutically effective amount" can encompass amounts that would improve the overall therapy, reduce or prevent a symptom or cause of a disorder, or enhance the therapeutic effectiveness of another therapeutic agent.

When used in the present specification, unless otherwise specified, a "prophylactically effective amount" of a compound is an amount that is sufficient to prevent a disorder or one or more symptoms associated with a disorder, or to prevent a recurrence thereof. The prophylactically effective amount of a compound means the amount of a therapeutic agent that is able, either alone or in combination with another agent, to provide a prophylactic effect in the prevention of a disorder. The term "prophylactically effective amount" can encompass amounts that would improve overall prevention, or enhance the prophylactic effectiveness of another prophylactic agent.

Sarcopenia is related to muscles, and therefore to muscle strength, energy metabolism, nervous system, and secretory organs related to muscles. In one embodiment, the sarcopenia-related disease, disorder, or symptom includes at least one selected from the group consisting of a disease, disorder, or symptom caused by impaired muscle strength; a disease, disorder, or symptom caused by impaired energy metabolism; a disease, disorder, or symptom caused by impaired nervous system; and a disease, disorder, or symptom caused by an impaired secretory organ. In some embodiments, the sarcopenia-related disease, disorder, or symptom includes primary sarcopenia, secondary sarcopenia, and associated diseases, disorders, or symptoms thereof. In other embodiments, the sarcopenia-related disease, disorder, or symptom includes at least one selected from the group consisting of sarcopenia-related lifestyle diseases (non-wasting diseases), wasting diseases, tumor-related diseases (including cancer cachexia), infectious diseases and related diseases such as COVID-19, locomotor diseases, neurodegenerative diseases (including ALS), cognitive impairments, and other related impairments (any impairment defined as including malnutrition, frailty, spinal and lung injury, inactivity, reduced activity, disuse syndrome, and invasion such as trauma and surgery). In other embodiments, the sarcopenia-related disease, disorder, or symptom is that of a system selected from the group consisting of digestive system (such as digestive tract), circulatory system, respiratory system (such as vocal organs), urinary system, reproductive system, endocrine system, sensory system, cerebral nervous system, and motor system (such as bones, joints, ligaments, and muscles).

In specific embodiments, examples of diseases, disorders, or symptoms caused by muscle strength impairment that could be regulated, delayed in its progression, prevented, or treated, include neurogenic muscular atrophy, myogenic muscular atrophy (such as muscular dystrophy, congenital myopathy, mitochondrial encephalomyopathy, myopathy due to congenital metabolic abnormality, and other myopathies), and the like.

In specific embodiments, examples of diseases, disorders, or symptoms caused by energy metabolism impairment that could be regulated, delayed in its progression, prevented, or treated, include diabetes, dyslipidemia, obesity, metabolic syndrome, osteoporosis, fatty liver, hyperuricemia, and hypertension.

In specific embodiments, examples of diseases, disorders, or symptoms caused by secretory organ impairment that could be regulated, delayed in its progression, prevented, or treated include diabetes, pancreatitis, ulcerative colitis, diarrhea, and the like.

In specific embodiments, examples of diseases, disorders, or symptoms caused by nervous system impairment that could be regulated, delayed in its progression, prevented, or treated include neurodegenerative diseases (including ALS), cognitive impairment, and the like.

In another embodiment, the sarcopenia-related diseases, disorders, or symptoms can be classified as primary or secondary.

The sarcopenia-related diseases, disorders, or symptoms include at least one selected from the group consisting of primary sarcopenia, secondary sarcopenia, and associated diseases, disorders, or symptoms thereof.

In specific embodiments, examples of diseases, disorders, or symptoms associated with primary sarcopenia and/or secondary sarcopenia that could be regulated, delayed in its progression, prevented, or treated include being bedridden, inactive lifestyle, poor health, severe organ failure, inflammatory diseases, malabsorption, gastrointestinal diseases, loss of appetite, and the like.

In specific embodiments, the compounds of the present invention may be selected from compounds represented by the above formula (1), and compounds represented by the above formula (2). In a preferred embodiment, the compounds of the present invention, or compounds, pharmaceutically acceptable salts, or solvates thereof can be orally administered. In another embodiment, the pharmaceutical composition of the present invention may be administered in combination with at least one of exercise therapy, diet therapy, and another drug therapy. In specific embodiments, the pharmaceutical composition of the present invention may be administered in combination with exercise therapy. In specific embodiments, the regulation, delay of progression, prevention, or treatment includes regulation, delay of progression, prevention, or treatment of at least one of: muscle mass loss, decreased (muscle) endurance, reduced short-term memory, bone density loss, and osteoporosis. In further specific embodiments, the regulation, delay of progression, prevention, or treatment can include regulation, delay of progression, prevention, or treatment in muscle mass loss and/or decreased (muscle) endurance.

The administration route of the compounds, pharmaceuticals, compositions, etc., of the present invention may be oral administration, parenteral administration, or rectal administration, and the daily dosage thereof varies depending on the type of compound, the administration method, the patient's symptoms, age, and the like. For example, in the case of oral administration, they are typically administered to a human or mammal in an amount of about 0.01 to 1000 mg per kilogram of body weight, and more preferably about 0.1 to 500 mg per kilogram of body weight, divided into one or several doses. In the case of parenteral administration, such as intravenous injection, they are typically administered to a human or mammal in an amount of about 0.01 to 300 mg per kilogram of body weight, and more preferably about 1 to 100 mg per kilogram of body weight.

Although the description of the pharmaceutical compositions provided in the present Specification mainly relates to pharmaceutical compositions for administration to humans, it will be appreciated by a person skilled in the art that said compositions are generally suitable for administration to all types of animals. Required modifications of pharmaceutical compositions for administration to various animals are well understood, and an ordinary veterinary pharmacologist would be able to design and/or perform such modifications with, at most, simple routine experimentation.

The compounds, pharmaceuticals, compositions, etc. of the present inventions can be administered orally or parenterally, either directly or after being formulated using a suitable dosage form. Examples of dosage forms include, but are not limited to, tablets, capsules, powders, granules, liquids, suspensions, injections, patches, and poultices. The formulations are manufactured by well-known methods using pharmaceutically acceptable excipients. Excipients that can be used, depending on the purpose, include excipients, disintegrants, binders, fluidizers, lubricants, coating agents, solubilizers, solubilizing agents, thickeners, dispersants, stabilizers, sweeteners, flavors, and the like. Specific examples include lactose, mannitol, crystalline cellulose, low-substituted hydroxypropyl cellulose, corn starch, partly pregelatinized starch, carmellose calcium, croscarmellose sodium, hydroxypropyl cellulose, hydroxypropyl methylcellulose, polyvinyl alcohol, magnesium stearate, sodium stearyl fumarate, polyethylene glycol, propylene glycol, titanium oxide, and talc.

As used in the present Specification, pharmaceutically acceptable excipients include any or all solvents, dispersion media, diluents, or other liquid carriers, dispersing or suspending aids, surfactants, isotonizing agents, thickening or emulsifying agents, preservatives, solid binders, lubricants, and the like, as appropriate for the specific dosage form that is desired. Remington's The Science and Practice of Pharmacy, 21St Edition, A. R. Gennaro, (Lipponcott, Williams & Wilkins, Baltimore, MD, 2006) discloses various excipients used for the formulation of pharmaceutical compositions and known techniques for the preparation thereof. Unless an excipient is incompatible with a substance that has a conventional carrier medium (for example, an undesirable biological effect is generated, or, interacts adversely with any other component(s) of the pharmaceutical composition), the use thereof is understood to be within the scope of the present invention.

In several embodiments, the pharmaceutically acceptable excipients are at least, 95%, 96%, 97%, 98%, 99%, or 100% pure. In several embodiments, the excipients are approved for human and veterinary use. In several embodiments, the excipients are approved by the U.S. Food and Drug Administration. In several embodiments, the excipients are of pharmaceutical grade. In several embodiments, the excipients meet the standards of the United States Pharmacopoeia (USP), the European Pharmacopoeia (EP), the British Pharmacopoeia, the Japanese Pharmacopoeia (JP), and/or the International Pharmacopoeia.

The pharmaceutically acceptable excipients used for the manufacture of the pharmaceutical compositions comprise, but are not limited to, inert diluents, dispersing and/or granulating agents, surfactants and/or emulsifying agents, disintegrants, binders, preservatives, buffers, lubricants, and/or oils. Said excipients may be optionally included in the formulations of the present invention. Additives such as cocoa butter and suppository waxes, coloring agents, coating agents, sweetening, flavoring, and perfuming agents can be present in the composition, in accordance with the judgment of the formulator.

Exemplary diluents include, but are not limited to, calcium carbonate, sodium carbonate, calcium phosphate, dicalcium phosphate, calcium sulfate, calcium hydrogen phosphate, sodium phosphate, lactose, sucrose, cellulose, microcrystalline cellulose, kaolin, mannitol, sorbitol, inositol, sodium chloride, dry starch, corn starch, powdered sugar, and combinations thereof.

Exemplary granulating and/or dispersing agents include, but are not limited to, potato starch, corn starch, tapioca starch, sodium starch glycolate, clays, alginic acid, guar gum, citrus pulp, agar, bentonite, cellulose and wood products, natural sponge, cation exchange resins, calcium carbonate, silicates, sodium carbonate, cross-linked polyvinylpyrrolidone (crospovidone), sodium carboxymethyl starch (sodium starch glycolate), carboxymethylcellulose, internally cross-linked sodium carboxymethylcellulose (croscarmellose), methylcellulose, gelatinized starch (starch 1500), microcrystalline starch, water insoluble starch, calcium carboxymethylcellulose, magnesium aluminum silicate (Veegum), sodium lauryl sulfate, quaternary ammonium compounds, and combinations thereof.

Exemplary surfactants and/or emulsifiers include, but are not limited to: natural emulsifiers (such as acacia, agar, alginic acid, sodium alginate, tragacanth, chondrux, cholesterol, xanthan, pectin, gelatin, egg yolk, casein, wool fat, cholesterol, waxes, and lecithin); colloidal clays (such as bentonite [aluminum silicate] and Veegum [aluminum magnesium silicate]); long-chain amino acid derivatives; high molecular weight alcohols (such as stearyl alcohol, cetyl alcohol, oleyl alcohol, triacetin monostearate, ethylene glycol distearate, glyceryl monostearate, propylene glycol monostearate, and polyvinyl alcohol); carbomers (such as carboxypolymethylene, polyacrylic acid, acrylic acid polymer, and carboxyvinyl polymer); carrageenan; cellulose derivatives (such as sodium carboxymethyl cellulose, powdered cellulose, hydroxymethyl cellulose, hydroxypropyl cellulose, hydroxypropyl methylcellulose, methylcellulose); sorbitan fatty acid esters (such as polyoxyethylene sorbitan monolaurate [Tween-20], polyoxyethylene sorbitan [Tween-60], polyoxyethylene sorbitan monooleate [Tween-80], sorbitan monopalmitate [Span-40], sorbitan monostearate [Span-60], sorbitan tristearate [Span-65], glyceryl monooleate, sorbitan monooleate [Span-80]); polyoxyethylene esters (such as polyoxyethylene monostearate [Myrj-45], polyoxyethylene hydrogenated castor oil, polyethoxylated castor oil, polyoxyethylene stearate, and Solutol); sucrose fatty acid esters; polyethylene glycol fatty acid esters (such as Cremophor); polyoxyethylene ethers (such as polyoxyethylene lauryl ether [Brij-30]); polyvinylpyrrolidone; diethylene glycol monolaurate; triethanolamine oleate; sodium oleate; potassium oleate; ethyl oleate; oleic acid; ethyl laurate; sodium lauryl sulfate; Pluronic^{®} F68; Poloxamer 188; cetrimonium bromide; cetylpyridinium chloride; benzalkonium chloride; docusate sodium; and combinations thereof.

Exemplary binders include, but are not limited to, starches (such as corn starch and starch paste), gelatin, sugars (such as sucrose, glucose, dextrose, dextrin, molasses, lactose, lactitol, mannitol), natural and synthetic gums (such as acacia, sodium alginate, Irish moss extract, panwar gum, ghatti gum, isapol husk mucilage, carboxymethylcellulose, methylcellulose, ethylcellulose, hydroxyethylcellulose, hydroxypropylcellulose, hydroxypropylmethylcellulose, microcrystalline cellulose, cellulose acetate, polyvinylpyrrolidone, magnesium aluminum silicate (Veegum), and larch arabogalactan), alginic acid, polyethylene oxide, polyethylene glycol, inorganic calcium salts, silicic acid, polymethacrylates, waxes, water, alcohol, and combinations thereof.

Exemplary preservatives may include antioxidants, chelating agents, antimicrobial preservatives, antifungal preservatives, alcohol preservatives, acidic preservatives, and other preservatives. Exemplary antioxidants include, but are not limited to, alpha tocopherol, ascorbic acid, acorbyl palmitate, butylated hydroxyanisole, butylated hydroxytoluene, monothioglycerol, potassium pyrosulfite, propionic acid, propyl gallate, sodium ascorbate, sodium bisulfite, sodium pyrosulfite, and sodium sulfite. Exemplary chelating agents include ethylenediaminetetraacetic acid (EDTA), citric acid monohydrate, edetate disodium, edetate dipotassium, edetate, fumaric acid, malic acid, phosphoric acid, sodium edetate, tartaric acid, and edetate trisodium. Exemplary antimicrobial preservatives include, but are not limited to, benzalkonium chloride, benzethonium chloride, benzyl alcohol, bronopol, cetrimide, cetylpyridinium chloride, chlorhexidine, chlorobutanol, chlorocresol, chloroxylenol, cresol, ethyl alcohol, glycerin, hexetidine, imidurea, phenol, phenoxyethanol, phenylethyl alcohol, phenylmercuric nitrate, propylene glycol, and thimerosal. Exemplary antifungal preservatives include, but are not limited to, butylparaben, methylparaben, ethylparaben, propylparaben, benzoic acid, hydroxybenzoic acid, potassium benzoate, potassium sorbate, sodium benzoate, sodium propionate, and sorbic acid. Exemplary alcohol preservatives include, but are not limited to, ethanol, polyethylene glycol, phenol, phenolic compounds, bisphenol, chlorobutanol, hydroxybenzoic acid, and phenylethyl alcohol. Exemplary acidic preservatives include, but are not limited to, vitamin A, vitamin C, vitamin E, beta-carotene, citric acid, acetic acid, dehydroacetic acid, ascorbic acid, sorbic acid, and phytic acid. Other preservatives include, but are not limited to, tocopherol, tocopherol acetate, deteroxime mesylate, cetrimide, butylated hydroxyanisole (BHA), butylated hydroxytoluened (BHT), ethylenediamine, sodium lauryl sulfate (SLS), sodium lauryl ether sulfate (SLES), sodium bisulfite, sodium pyrosulfite, potassium sulfite, potassium pyrosulfite, Glydant Plus, Phenonip, methylparaben, Germall 115, Germaben II, Neolone, Kathon, and Euxyl. In some embodiments, the preservative is an antioxidant. In other embodiments, the preservative is a chelating agent.

Exemplary buffering agents include, but are not limited to, citrate buffer solution, acetate buffer solution, phosphate buffer solution, ammonium chloride, calcium carbonate, calcium chloride, calcium citrate, calcium glubionate, calcium glucoheptonate, calcium gluconate, D-gluconic acid, calcium glycerophosphate, calcium lactate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, magnesium hydroxide, aluminum hydroxide, alginic acid, pyrogen-free water, isotonic saline, Ringer's solution, ethyl alcohol, and combinations thereof.

Exemplary lubricants include, but are not limited to, magnesium stearate, calcium stearate, stearic acid, silica, talc, malt, glyceryl behanate, hydrogenated vegetable oils, polyethylene glycol, sodium benzoate, sodium acetate, sodium chloride, leucine, magnesium lauryl sulfate, sodium lauryl sulfate, and combinations thereof.

Exemplary oils include, but are not limited to, almond, apricot kernel, avocado, babassu, bergamot, blackcurrant seed, borage, cade, chamomile, canola, caraway, carnauba, castor, cinnamon, cocoa butter, coconut, cod liver, coffee, corn, cottonseed, emu, eucalyptus, evening primrose, fish, flaxseed, geraniol, gourd, grape seed, hazelnut, hyssop, isopropyl myristate, jojoba, kukui nut, lavandin, lavender, lemon, litsea cubeba, macadamia nut, mallow, mango seed, meadowfoam seed, mink, nutmeg, olive, orange, orange roughy, palm, palm kernel, peach kernel, peanut, poppy seed, pumpkin seed, rapeseed, rice bran, rosemary, safflower, sandalwood, sasanqua, spearmint, sea buckthorn, sesame, shea butter, silicone, soybean, sunflower, tea tree, thistle, tsubaki, vetiver, walnut, and wheat germ oil. Exemplary oils include, but are not limited to, butyl stearate, caprylic triglyceride, capric triglyceride, cyclomethicone, diethyl sebacate, dimethicone 360, isopropyl myristate, mineral oil, octyldodecanol, oleyl alcohol, silicone oil, and combinations thereof.

Liquid dosage forms for oral and parenteral administration include, but are not limited to, pharmaceutically acceptable emulsions, microemulsions, solutions, suspensions, syrups, and elixirs. In addition to active ingredients, the liquid dosage forms may contain inert diluents commonly used in the present field, such as water or other solvents, and solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, dimethylformamide, oils (especially cottonseed, peanut, corn, germ, olive, castor, and sesame oils), glycerol, tetrahydrofurfuryl alcohol, polyethylene glycols, fatty acid esters of sorbitan, and mixtures thereof. In addition to inert diluents, oral compositions can include adjuvants, such as wetting agents, emulsifying and suspending agents, sweetening, flavoring, and perfuming agents. In some embodiments for parenteral administration, complexes of the present invention are admixed with a solubilizing agent, such as cremophor, alcohol, oil, modified oil, glycol, polysorbate, cyclodextrin, polymer, or combinations thereof.

Injectable preparations, for example, sterile injectable aqueous or oleaginous suspensions, can be formulated in accordance with the known art using dispersing or wetting agents and suspending agents. The sterile injectable preparation may also be a sterile injectable solution, suspension, or emulsion in a non-toxic parenterally acceptable diluent or solvent, for example a solution in 1,3-butanediol. Acceptable vehicles and solvents that may be employed are water, Ringer's solution (USP) and isotonic sodium chloride solution. In addition, sterile, non-volatile oils are conventionally used as a solvent or a suspending medium. For this purpose, any non-irritating and non-volatile oil can be used, including synthetic mono- or diglycerides. In addition, fatty acids such as oleic acid can be used in the preparation of injectables.

The injectable preparations can be sterilized, for example, by means of filtration through a bacterial-retaining filter, or by incorporating sterilizing agents in the form of sterile solid compositions (which can be dissolved or dispersed in sterile water or other sterile injectable medium prior to use).

In order to prolong the effect of a drug, it is often desirable to slow the absorption of the drug after subcutaneous or intramuscular injection. This can be accomplished by using a crystalline or non-crystalline liquid suspension having poor water solubility. The rate of absorption of a drug then depends on the rate of dissolution thereof, which, in turn, may depend on the crystal size and crystalline form. In contrast, delayed absorption of a parenterally administered drug form can be achieved by dissolving or suspending the drug in an oil base.

Compositions for rectal or vaginal administration are typically suppositories, which can be prepared by admixing a complex of the present invention with a non-irritating excipient or carrier (such as cocoa butter, polyethylene glycol, or a suppository wax). These compositions are solid at room temperature but liquid at body temperature, and thus melt in the rectal or vaginal cavity releasing the active ingredient.

Solid dosage forms for oral administration include capsules, tablets, pills, powders, and granules. In such solid dosage forms, the active ingredient is admixed with at least one inert, pharmaceutically acceptable excipient or carrier. Examples thereof include sodium citrate or dicalcium phosphate, and/or (a) fillers or extenders, such as starches, lactose, sucrose, glucose, mannitol, and silicic acid; (b) binders, such as carboxymethylcellulose, alginic acid, gelatin, polyvinylpyrrolidinone, sucrose, and acacia; (c) humectants, such as glycerol; (d) disintegrating agents, such as agar, calcium carbonate, potato or tapioca starch, alginic acid, certain silicates, and sodium carbonate; (e) solution retarders, such as paraffin; (f) absorption accelerators, such as quaternary ammonium compounds; (g) wetting agents, such as cetyl alcohol and glycerol monostearate; (h) adsorbents, such as kaolin and bentonite clays; and (i) lubricants, such as talc, calcium stearate, magnesium stearate, solid polyethylene glycols, sodium lauryl sulfate, and mixtures thereof. In the case of capsules, tablets, and pills, the dosage form may include buffering agents.

Solid compositions of a similar type can also be used as fillers in soft and hard filled gelatin capsules using excipients such as lactose or milk sugar as well as high-molecular-weight polyethylene glycols and the like. The solid dosage forms of tablets, sugar-coated tablets, capsules, pills, and granules can be prepared with coatings and shells such as enteric coatings and other coatings well known in the field of pharmaceutical formulations. The coatings may optionally contain opacifying agents and may be compositions that release the active ingredient(s) only, or preferentially, in a certain part of the intestinal tract, in a delayed release manner. Examples of embedding compositions that can be used include polymeric substances and waxes. Solid compositions of a similar type can also be used as fillers in soft and hard filled gelatin capsules using excipients such as lactose or milk sugar as well as high-molecular-weight polyethylene glycols and the like. Solid compositions of a similar type can also be used as fillers in soft and hard filled gelatin capsules using excipients such as lactose or milk sugar as well as high-molecular-weight polyethylene glycols and the like.

The active ingredient can be in a microencapsulated form, containing one or more of the excipients described above. The solid dosage forms of tablets, sugar-coated tablets, capsules, pills, and granules can be prepared with coatings and shells such as enteric coatings, controlled-release coatings, and other coatings well known in the field of pharmaceutical formulations. In such solid dosage forms, the active ingredient may be admixed with at least one inert diluent such as sucrose, lactose, or starch. Such dosage forms may also comprise, in the usual course of practice, additional substances other than inert diluents, such as tableting lubricants and other tableting aids, including magnesium stearate and microcrystalline cellulose. In the case of capsules, tablets, and pills, the dosage form may include buffering agents. The coatings may optionally contain opacifying agents and may be compositions that release the active ingredient(s) only, or preferentially, in a certain part of the intestinal tract, in a delayed release manner. Examples of embedding compositions that can be used include polymeric substances and waxes. Solid compositions of a similar type can also be used as fillers in soft and hard filled gelatin capsules using excipients such as lactose or milk sugar as well as high-molecular-weight polyethylene glycols and the like.

Dosage forms for topical and/or transdermal administration of the compounds, salts, solvates, and compositions of the present invention may include ointments, pastes, creams, lotions, gels, powders, solutions, sprays, inhalants, and/or patches. In general, active ingredients can be admixed in a sterile state with a pharmaceutically acceptable carrier and/or any required preservative and/or buffering agent, as may be required. In addition, usage of transdermal patches is also considered in the present invention, which, in many cases, has the additional advantage of providing controlled delivery of active ingredients to the body. Such a dosage form can be prepared, for example, by dissolving and/or compounding an active ingredient into an appropriate medium. Alternatively, or additionally, the speed can be controlled by providing a rate-controlling membrane and/or by dispersing the active ingredient in a polymer matrix and/or gel.

Suitable devices for use in delivering the intradermal pharmaceutical compositions described in the present Specification include short needle devices, such as those described in U.S. Patents 4,886,499, 5,190,521, 5,328,483, 5,527,288, 4,270,537, 5,015,235, 5,141,496, and 5,417,662. Intradermal compositions can be administered using devices that limit the effective penetration length of the needle into the skin, such as that described in PCT Publication No. WO99/34850 and functional equivalents thereof. Jet injection devices are described, for example, in U.S. Patents 5,480,381, 5,599,302, 5,334,144, 5,993,412, 5,649,912, 5,569,189, 5,704,911, 5,383,851, 5,893,397, 5,466,220, 5,339,163, 5,312,335, 5,503,627, 5,064,413, 5,520,639, 4,596,556, 4,790,824, 4,941,880, 4,940,460, and PCT publication No. WO 97/37705 and No. WO 97/13537. Alternatively or additionally, conventional syringes may be used to perform the classical Mantoux technique of intradermal administration.

Formulations for topical administration include, but are not limited to, liquid and/or semi-liquid preparations such as liniments, lotions, oil-in-water and/or water-in-oil emulsions, such as creams, ointments, and/or pastes, and/or solutions and/or suspensions. Formulations that can be administered topically may contain from about 1% to about 10% (w/w) of the active ingredient, for example, but the concentration of the active ingredient may be as high as the solubility limit of the active ingredient in the solvent. Formulations for topical administration may further comprise one or more additional ingredients described in the present Specification.

The pharmaceutical composition of the present invention may be prepared, packaged, and/or sold as a formulation for pulmonary administration via the buccal cavity. Such formulations may comprise dry particles that contain the active ingredient and has a particle diameter in the range from about 0.5 to about 7 nanometers, or from about 1 to about 6 nanometers. Such compositions are conveniently in the form of dry powder for the purpose of administration using a device that includes a dry powder reservoir (into which a propellant stream can be directed to disperse the powder) and/or using a self-propelling solvent/powder dispenser container (such as a device containing the active ingredient dissolved and/or suspended in a low-boiling propellant in a sealed container). Such powders comprise particles in which at least 98% of the particles by weight have a diameter greater than 0.5 nanometers and at least 95% of the particles by number have a diameter less than 7 nanometers. Alternatively, such powders comprise particles in which at least 95% of the particles by weight have a diameter greater than 1 nanometer and at least 90% of the particles by number have a diameter less than 6 nanometers. Dry powder compositions may include a solid fine powder diluent, such as sugar, and are conveniently provided in a unit dose form.

Low-boiling propellants generally comprise a liquid propellant having a boiling point of less than 65°F at atmospheric pressure. In general, the propellant may constitute 50-99.9% (w/w) of the composition, and the active ingredient may constitute 0.1 = 20% (w/w) of the composition. The propellant may further comprise additional ingredients, such as a liquid nonionic and/or solid anionic surfactant and/or a solid diluent (which may have a particle size of the same order as the particles comprising the active ingredient).

The pharmaceutical compositions of the present invention formulated for pulmonary delivery can provide the active ingredient in the form of droplets of a solution and/or suspension. Such formulations can be prepared, packaged, and/or sold as aqueous and/or dilute alcoholic, optionally sterile, solutions and/or suspensions containing the active ingredient and may be conveniently administered using any nebulization and/or atomization device. Such formulations may further comprise one or more additional ingredients, which may include, but are not limited to, flavorings, such as sodium saccharin, volatile oils, buffers, surfactants, and/or preservatives, such as methyl hydroxybenzoate. The droplets provided by this route of administration may have an average diameter in the range from about 0.1 to about 200 nanometers.

The formulations described in the present Specification as being useful for pulmonary delivery are useful for intranasal delivery of the pharmaceutical compositions of the present invention. Another formulation for intranasal administration is a coarse powder, comprising the active ingredient and having an average particle size of about 0.2 to 500 micrometers. Such a formulation is administered in the manner in which snuff tobacco is taken, that is, by rapid inhalation through the nasal passage from a container of the powder held close to the nostril.

Formulations for nasal administration may contain, for example, from about 0.1 to 100% (w/w) of the active ingredient, and may include one or more of the additional ingredients described in the present Specification. The pharmaceutical composition of the present invention may be prepared, packaged, and/or sold as a formulation for buccal administration. Such formulations may, for example, be in the form of tablets and/or lozenges manufactured using conventional methods and may contain, for example, 0.1-20% (w/w) of the active ingredient, the remainder comprising an orally dissolvable and/or degradable composition and, optionally, one or more of the additional ingredients described in the present Specification. Alternatively, formulations for buccal administration may comprise a powder and/or an aerosolized and/or atomized solution and/or suspension comprising the active ingredient. Such powdered, aerosolized and/or aerosolized formulations, when dispersed, may have an average particle and/or droplet size in the range of about 0.1 to about 200 nanometers, and may further comprise one or more of the additional ingredients described in the present Specification.

The pharmaceutical composition of the present invention may be prepared, packaged, and/or sold as a formulation for ocular administration. Such formulations may, for example, be in the form of eye drops and include, for example, a 0.1/1.0% (w/w) solution and/or suspension of the active ingredient in an aqueous or oleaginous liquid carrier. Such drops may further comprise buffering agents, salt, and/or one or more other additional ingredients described in the present Specification. Other effective formulations that can be used for ocular administration include those that contain the active ingredient in microcrystalline form and/or in a liposomal formulation. Ear drops and/or eye drops are considered to be within the scope of the present invention.

General considerations in the formulation and/or manufacture of pharmaceutical agents can be found, for example, in Remington: The Science and Practice of Pharmacy 21st ed., Lippincott Wilkins, 2005.

In several embodiments, the pharmaceutical compositions of the present invention may be administered to a patient for at least two weeks, at least three weeks, at least four weeks, at least five weeks, at least six weeks, at least seven weeks, or at least eight weeks. In specific embodiments, the pharmaceutical composition of the present invention may be administered to a patient for at least four weeks. Typically the effects of sarcopenia can be evaluated in about four weeks.

The pharmaceutical compositions described in the present Specification may be prepared by any method known or hereafter developed in the field of pharmacology. In general, such preparatory methods include the step of bringing the active ingredient into association with an excipient and/or one or more other accessory ingredients, and then, if necessary and/or desirable, shaping and/or packaging the product into a desired single- or multi-dose unit.

The pharmaceutical composition of the present invention may be prepared, packaged, and/or sold in bulk, as a single unit dose, and/or as a plurality of single unit doses. As used herein, a "unit dose" is a discrete amount of the pharmaceutical composition comprising a predetermined amount of the active ingredient. The amount of the active ingredient is generally equal to the dosage of the active ingredient which would be administered to a subject and/or a convenient fraction of such a dosage (such as, for example, one-half or one-third of such a dosage). Relative amounts of the active ingredient, the pharmaceutically acceptable excipient, and/or any additional ingredients in a pharmaceutical composition of the present invention will vary, depending upon the identity, size, and/or condition of the subject to be treated and further depending upon the route by which the composition is to be administered. For example, the composition may comprise between 0.1% and 100% (w/w) active ingredient.

The compounds of the present invention are typically formulated in dosage unit form for ease of administration and uniformity of dosage. It will be understood, however, that the total daily usage of the pharmaceutical compositions of the present invention will be decided by the attending physician within the scope of sound medical judgment. The specific therapeutically effective dose level for any particular subject will depend on a variety of factors. Such factors include the disease, disorder, or disorder being treated and the severity of the disorder; the activity of the specific active ingredient employed; the age, body weight, general health, sex and diet of the subject; the time of administration, route of administration, and rate of excretion of the specific active ingredient employed; the duration of the treatment; drugs used in combination or simultaneously with the specific active ingredient employed; and like factors well known in the medical field.

The compounds of the present invention, pharmaceutically acceptable salts thereof, solvates thereof, pharmaceuticals, and the like, may be administered via any route. In several embodiments, a compound represented by the above formula (1), a compound represented by the above formula (2), a pharmaceutically acceptable salt, or a solvate thereof, or a pharmaceutical composition that comprises at least one of the foregoing is administered by various routes (including oral, intravenous, intramuscular, intra-arterial, intramedullary, intraspinal, subcutaneous, intraventricular, transdermal, intradermal, rectal, intravaginal, intraperitoneal, topical (such as by powder, ointment, cream, and/or drops), mucosal, nasal, buccal, enteral, sublingual, intratracheal infusion, intrabronchial infusion, and/or by inhalation, and/or as an oral spray, nasal spray, and/or aerosol). Specifically conceivable routes are systemic intravenous injection, local administration by means of blood transfusion and/or lymphatic infusion, and/or direct administration to the affected area. In general, the most appropriate administration route depends on various factors. Such factors include the properties of the agent (such as the stability thereof in the environment of the gastrointestinal tract) and/or the disorder of the subject (such as whether the subject is able to tolerate oral administration). Currently, oral and/or nasal spray and/or aerosol routes are most commonly used to deliver therapeutic agents directly to the lungs and/or respiratory system. However, the present invention takes into consideration possible advances in the science of drug delivery and encompasses delivery of the pharmaceutical compositions of the present invention by any suitable route.

The compounds, salts, solvates, and pharmaceutical compositions of the present invention may be administered at a dosage level sufficient to obtain the desired therapeutic effect, delivering about 0.001 mg/kg to about 100 mg/kg, about 0.01 mg/kg to about 50 mg/kg, about 0.1 mg/kg to about 40 mg/kg, about 0.5 mg/kg to about 30 mg/kg, about 0.01 mg/kg to about 10 mg/kg, about 0.1 mg/kg to about 10 mg/kg, or about 1 mg/kg to about 25 mg/kg of the subject's body weight, once or more times per day. The desired dosage may be delivered three times a day, two times a day, once a day, every other day, every third day, every week, every two weeks, every three weeks, or every four weeks. In certain embodiments, the desired dosage may be delivered using multiple administrations (for example, two, three, four, five, six, seven, eight, nine, ten, eleven, twelve, thirteen, fourteen, fifteen or more administrations).

It will be appreciated that the dosage ranges provided in the present Specification provide guidance for administration of the provided pharmaceutical compositions to adults. For example, the dose administered to a child or adolescent can be determined by a healthcare professional or a person skilled in the art, and can be lower than or the same as that administered to an adult. The exact amount of the compounds, salts, solvates, and compositions of the present invention required to achieve the effective amount will vary from subject to subject, depending, for example, on the subject's species, age, general disorder, the severity of side effects or the disorder, the identity of the specific compound(s), the mode of administration, and the like.

The present invention encompasses "therapeutic cocktails" comprising the compounds, salts, solvates, and pharmaceutical compositions of the present invention. In several embodiments, the compounds, salts, solvates, and pharmaceutical compositions of the present invention comprise a single type that can bind to a plurality of targets. In several embodiments, different compounds, salts, solvates, and pharmaceutical compositions of the present invention comprise different targeting moiety species, and all of the different targeting moiety species can bind to the same target. In several embodiments, different compounds, salts, solvates, and pharmaceutical compositions of the present invention comprise different targeting moiety species, and all of the different targeting moiety species can bind to different targets. In several embodiments, said different targets may be associated with the same cell type. In several embodiments, said different targets may be associated with different cell types.

It will be appreciated that the compounds, salts, solvates, and pharmaceutical compositions of the present invention may be used in combination therapy. Specific combinations of therapies (therapeutic agents or procedures) for using in a combination regimen may be appropriately determined in consideration of compatibility of the desired therapeutic agents and/or procedures and the desired therapeutic effect to be achieved. Naturally, the therapies to be used may achieve the desired effect for the same purpose (for example, a complex of the present invention that is useful for detecting tumors may be administered at the same time as another agent that is useful for detecting tumors), or achieve different effects (for example, controlling any of the side effects).

The pharmaceutical compositions of the present invention can be administered alone or in combination with one or more therapeutically active agents. The phrase "in combination with" is not intended to mean that the agents must necessarily be formulated for simultaneous administration and/or to be delivered together, but the methods of delivery are within the scope of the present invention. The compositions may be administered before, after, or at the same time as one or more other desired therapeutic agents or medical treatments. In general, each agent may be administered at the dose and/or time schedule determined for that agent. In addition, the present invention encompasses the delivery of the pharmaceutical compositions of the present invention in combination with agents that may improve their bioavailability, reduce and/or modify their metabolism, inhibit their excretion, and/or modify their distribution within the body. It will further be appreciated that the therapeutically active agents and the compounds, salts, solvates, or pharmaceutical compositions of the present invention used in this combination may be administered together as a single composition or administered separately as different compositions.

The specific combination used in the combination regimen will take into account the compatibility of the therapeutically active agent and/or treatment with the compound, salt, solvate, pharmaceutical composition of the present invention and/or the desired therapeutic effect to be achieved. It will be appreciated that the combinations to be used may achieve the desired effect for the same disorder (for example, a compound, salt, solvate, or pharmaceutical composition of the present invention may be administered at the same time as another therapeutically active agent used to treat the same disorder) and/or achieve a different effect (for example, controlling any of the side effects).

As used in the present Specification, "therapeutically active agent" refers to any substance used as a pharmaceutical for the treatment, prevention, delay, reduction, or amelioration of a disorder, and refers to a substance useful for therapies including prophylactic and therapeutic treatments. The therapeutically active agents also include compounds that enhance the effects of the compounds, salts, solvates, or pharmaceutical compositions of the present invention or reduces the side effects, thereby increasing the effect or efficacy of another compound.

In some embodiments, the therapeutically active agent is an anticancer agent, an antibiotic, an antiviral agent, an anti-HIV agent, an anti-parasitic agent, an anti-protozoal agent, an anesthetic, an anticoagulant, an enzyme inhibitor, a steroid, a steroidal or non-steroidal anti-inflammatory drug, an antihistamine, an immunosuppressant, an anti-tumor agent, an antigen, a vaccine, an antibody, a decongestant, a sedative, an opioid, an analgesic, an antipyretic, a contraceptive, a hormone, a prostaglandin, a progesterone agonist, an anti-glaucoma drug, an ophthalmic drug, an anticholinergic drug, an analgesic, an antidepressant, an antipsychotic, a neurotoxin, a hypnotic, a tranquilizer, an anticonvulsant, a muscle relaxant, an antiparkinsonian, an antispasmodic, a muscle contractant, a channel blocker, miotics, an antisecretory agent, an antithrombotic agent, an anticoagulant, an anticholinergic agent, a beta-adrenergic blocking agent, a diuretic, a cardiovascular agent, a vasoactive agent, a vasodilator, an antihypertensive agent, an angiogenic agent, a modulator of cell-extracellular matrix interactions (such as a cell proliferation inhibitor and an anti-adhesion molecule), or an inhibitor/intercalator of DNA, RNA, protein-protein interactions, or protein-receptor interactions.

The invention provides various kits including one or more of the compounds, salts, solvates, and pharmaceutical compositions of the present invention. For example, the present invention provides a kit comprising a compound, a salt, a solvate, or a pharmaceutical composition of the present invention and instructions for use. The kit may comprise a plurality of different compounds, salts, solvates, and pharmaceutical compositions of the present invention. The kit may comprise any of several additional ingredients or reagents in any combination. While not all of the various combinations are explicitly illustrated, each combination is within the scope of the present invention.

In accordance with certain aspects of the present invention, the kit may include, for example, (i) one or more of the compounds, salts, solvates, and pharmaceutical compositions of the present invention to be delivered, and optionally one or more specific therapeutically active agents, and (ii) instructions for administration to a subject in need thereof.

Kits typically include instructions, which may include the protocol, for example, and/or describe the production of the compounds, salts, solvates, and pharmaceutical compositions of the present invention, administration of the compounds, salts, solvates, and pharmaceutical compositions of the present invention to a subject in need thereof, and diseases, disorders, or symptoms for which the compounds, salts, solvates, and pharmaceutical compositions of the present invention were newly designed. The kits generally include one or more vessels or containers, which may accommodate some of all of the individual ingredients and reagents separately. The kits may also include a means, such as a plastic box, for packaging the individual containers relatively densely for commercial sale. Instructions and packing material, such as Styrofoam^{®}, may be contained therein. An identifier, such as a bar code, a radio frequency identification (ID) tag, or the like, may be present in or on the kit, or on one or more of the vessels or containers contained in the kit. The identifier may be used to uniquely identify the kit, for example, for purposes of quality control, inventory control, tracking, and transfer between workstations.

In the present Specification, the term "or" is used when "at least one or more" of the items listed in the sentence can be employed. The same goes for "or." When it is clearly stated in the present Specification that a range is "between two values," the range includes the two values themselves. In addition, all references cited in the present Specification, including scientific literature, patents, patent applications, and the like, are hereby incorporated by reference in their entirety to the same extent as if each were specifically set forth.

The present invention has been described above by illustrating preferred embodiments for ease of understanding. The present invention will be described below based on examples, but the descriptions described above and the following examples are provided for illustrative purposes only, and are not intended to limit the present invention. Accordingly, the scope of the present invention is not limited to the embodiments or examples specifically described in the present Specification, but is limited only by the claims.

### Examples

### [Example 1: L-ethylcysteine hydrochloride]

### (Example 1-1: Effects of the compound and effects of combination with exercise therapy)

In the present example, it was confirmed whether various sarcopenia-related diseases, disorders, or symptoms could be treated using L-ethylcysteine hydrochloride represented by formula (1) in the following model mice.

### Decreased endurance in aged mice

To confirm that muscle strength decline would be observed in model mice (C57BL/6J) (purchased from ORIENTAL YEAST), muscle strength (endurance) in 79-week-old aged mice was checked in a treadmill test as follows.

Before recording performance, the mice were trained for three days so as to become familiarized with the equipment. An electrical stimulation grid was adjusted to 1 mA and the gradient was set to 15 degrees. On the first day of training, the mice were made to walk on the treadmill for 10 min at a speed of 10 m/min, rest for 10 min, and then walk for 10 min at a speed of 10 m/min. On the second and third days, the first two steps were performed under the same conditions as on the first day, after which walking was started at 10 m/min, and then increasing the speed by 1 m/min each minute up to a maximum speed of 20 m/min. Maximum exercise endurance was measured on the fourth day. Six mice were placed on the treadmill and the belt speed was started at 5 m/min for 5 min to warm up the mice. The speed was increased by 1 m/min to a maximum of 20 m/min. After 5 minutes of running, the speed was increased from 20 m/min to 21 m/min for 10 minutes. The mice were then forced to run at 22 m/min until they remained on the electrical stimulation grid for 10 seconds, and the distance they ran until they remained for 10 seconds was measured.

As a result, in the treadmill test, the traveled distance of the mice, or their endurance, decreased significantly with age.

### Muscle strength increase in aged mice by means of administration of L-ethylcysteine hydrochloride

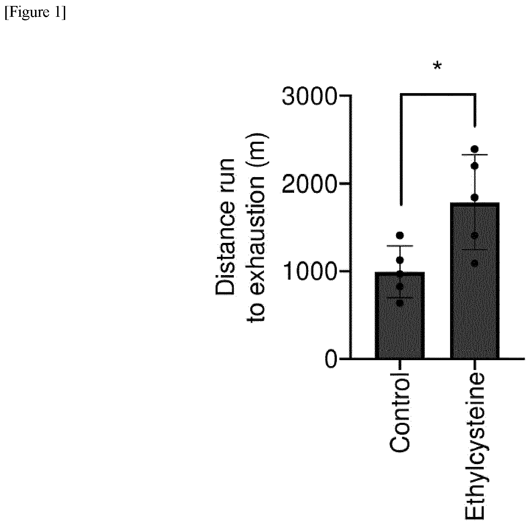

L-ethylcysteine hydrochloride was orally administered to aged mice from 40 to 79 weeks of age. The dose was 1000 mg/kg/day. During the administration period, no significant changes in body weight or behavioral abnormalities were observed. After administration of L-ethylcysteine hydrochloride at 79 weeks of age, muscle strength (endurance) was measured by the treadmill test; as a result, an increase in muscle strength by means of administration of L-ethylcysteine hydrochloride was observed specifically in aged mice (Figure 1).

As a result, it was observed that administration of L-ethylcysteine hydrochloride increased muscle mass in aged mice, and that the effect was maximized when aged mice that were administered L-ethylcysteine hydrochloride were made to exercise. This result indicates that L-ethylcysteine hydrochloride increases muscle strength in aged mice. It was also shown that administration of L-ethylcysteine hydrochloride in combination with exercise significantly restored muscle mass. The foregoing indicates that L-ethylcysteine hydrochloride may be useful as a therapeutic agent for sarcopenia.

### (Example 1-2: Alteration in gene expression in muscle by means of administration of L-ethylcysteine hydrochloride)

In the present example, gene regulation involved in the L-ethylcysteine hydrochloride of the present invention was investigated using model mice to identify genes useful for the diagnosis and treatment of sarcopenia.

### Treatment of sarcopenia by means of L-ethylcysteine hydrochloride administration

Genetic analysis (RNA-seq) was performed on aged mice administered with L-ethylcysteine hydrochloride, which showed improved (gastrocnemius or soleus) muscle strength and muscle mass. The procedure and reagents used are as follows. The quality of all reads was checked using FastQC v0.11.8 and low-quality reads were removed using Trimmomatic (ver 0.38).

The filtering conditions are as follows:
LEADING: 20 (cut bases off the start of a read if quality score is less than 20), TRAILING: 20, SLIDING WINDOW: 4:15 (window size 4, average quality 15), MINLEN: 36 (drop reads if the length is less than 3 bases)

Each read obtained after filtering was mapped to the reference genome GRCm38 using STAR (ver2.7.0.f). Mapping was performed using the default values of STAR. Since the reads were paired-end with a length of 150 bp, a STAR index for reference genome GRCm38 was created in accordance with the length of 150 to perform mapping.

Index generation was performed as follows:
Run Mode; genomeGenerate
Reference; GRCm38
Annotation file; genecode version M24
sjdbOverhang; 150
alignment was performed using the index generated above.
Other than the foregoing, no options were added and the default values were used.
(Basic options were as follows.)
genomeDir; index generated above

Furthermore, featureCounts (ver. 1.6.4) was used to calculate the count number for each gene name defined by the symbol name. At this time, gene symbols as defined in genecode version M24 were counted. Regarding the calculated count numbers, genes with count data of 0 in all samples were filtered, TMM normalization was performed using edgeR (ver3.22.3), and correction between samples was performed. First, to obtain an overview of the data, principal component analysis and clustering analysis were performed using the count data after removing low-expressing genes.

### Detection of differentially expressed genes

Expression changes of the specified patterns were compared based on the obtained expression values.

edgeR was used to test for differentially expressed genes. As for the definition of the differentially expressed genes, uncorrected p.value < 0.05 was used as the cutoff value, and "not used pattern" and "greater than or equal to 2 or less than or equal to 0.5" was used as the cutoff for fold change, to perform the calculations.

Gene enrichment analysis was performed on the obtained variable genes using gprofiler2 (version 0.1.6).

The expression values of the obtained differentially expressed genes were normalized, then extracted and visually depicted as a heat map to confirm the differential expressions. Transcription factor binding motif enrichment analysis was performed separately for up-regulated and down-regulated genes. This analysis was performed using RcisTarget (ver 1.8.0). In RcisTarget, analysis was performed using a mouse database relating to 10 kbp upstream the TSS, stored in RcisTarget. Details of the analysis are as follows (cited from https://scenic.aertslab.org/tutorials/RcisTarget.html).

As a first step to estimate the over-representation of each motif on the gene-set, the area under the curve (AUC) was calculated for each pair of motif-gene Set. This was calculated based on the recovery curve of the gene-set on the motif ranking (genes ranked in descending order by the score of motif in its proximity, as provided in the motifRanking database).

The selection of significant motifs was done based on the Normalized Enrichment Score (NES), and the NES was calculated for each motif based on the AUC distribution of all the motifs for the gene-set [(x-mean)/sd]. Those motifs that passed the given threshold (3.0 by default) were considered significant. Since RcisTarget searches for enrichment of a motif within a gene list, finding a motif "enriched" does not imply that all the genes in the gene list have a high score for the motif. In this way, the third step of the workflow was to identify which genes (of the gene-set) are highly ranked for each of the significant motifs.
These genes were identified using a faster implementation based on an approximate distribution using the average at each rank.

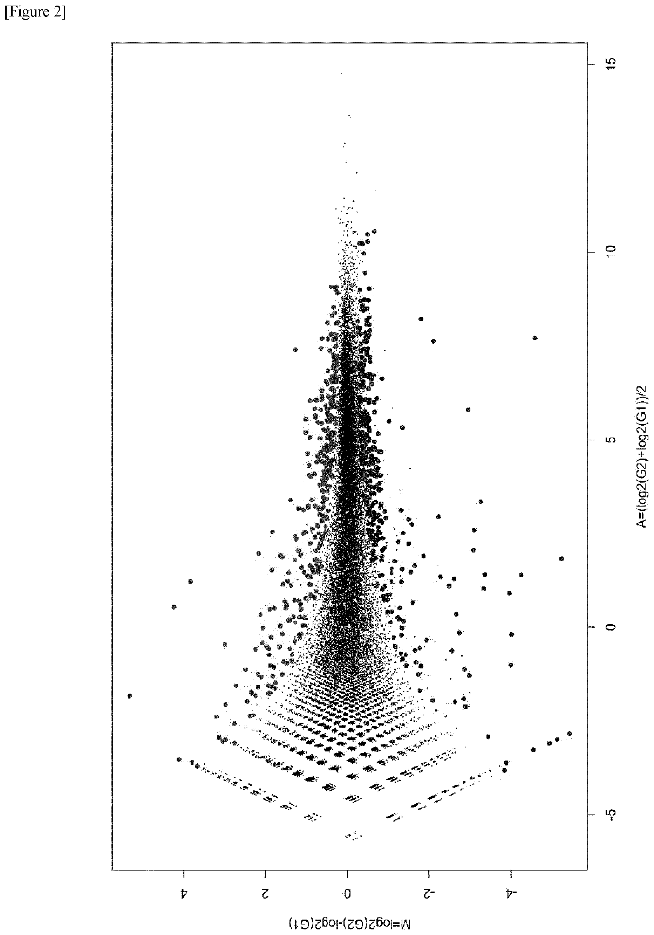

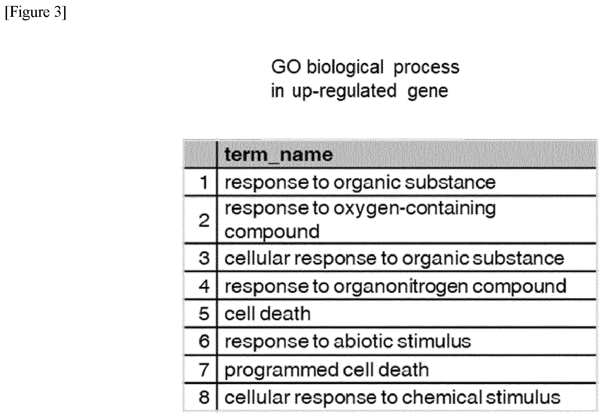

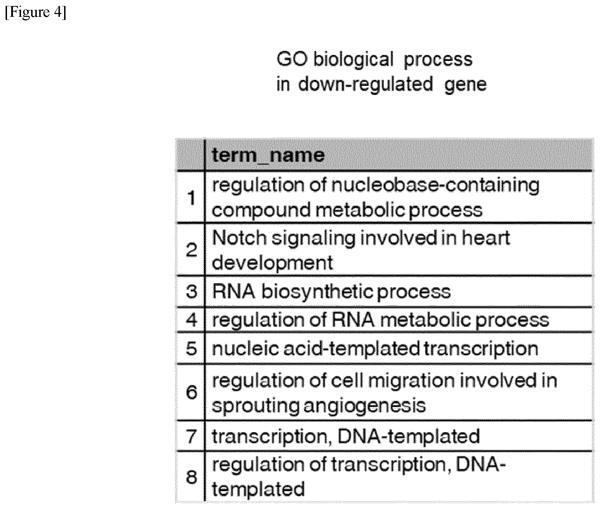

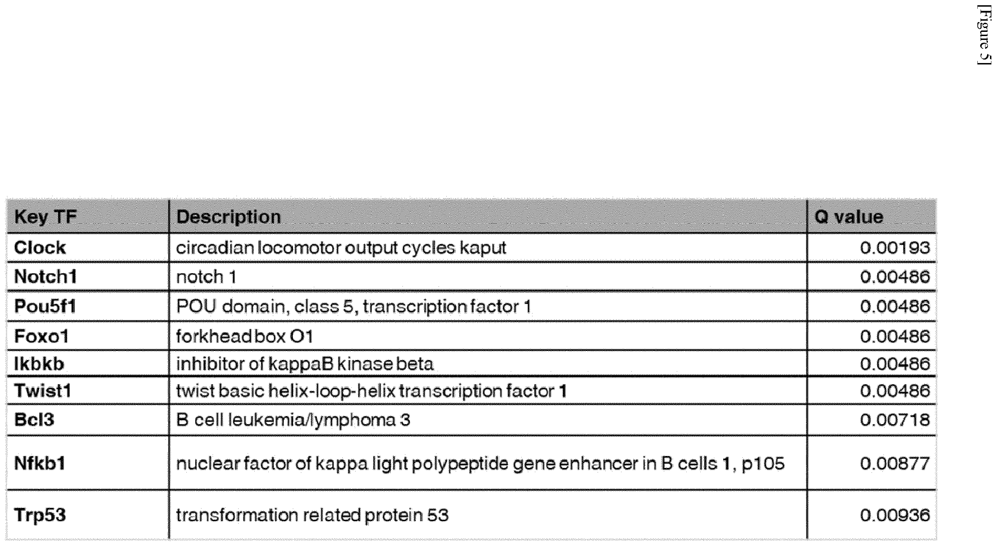

The results are shown in Figures 2 to 5. Administration of L-ethylcysteine hydrochloride increased expression of 158 genes and significantly decreased expression of 92 genes (Figure 2), and changes in gene clusters related to intercellular signals and nerves were observed (Figures 3 to 5). Figures 3 to 5 show results of pathway analysis of genes with increased expression, pathway analysis of genes with decreased expression, and analysis of upstream transcription factors of genes with altered expression, respectively. From the analysis of upstream transcription factors shown in Figure 5, new transcription factors involved in differentiation and cellular senescence were observed as upstream factors. This result suggests the possibility of developing diagnostic and therapeutic agents for sarcopenia using new targets.

### (Example 1-3: Disease and differentially expressed genes of muscle by means of administration of L-ethylcysteine hydrochloride)

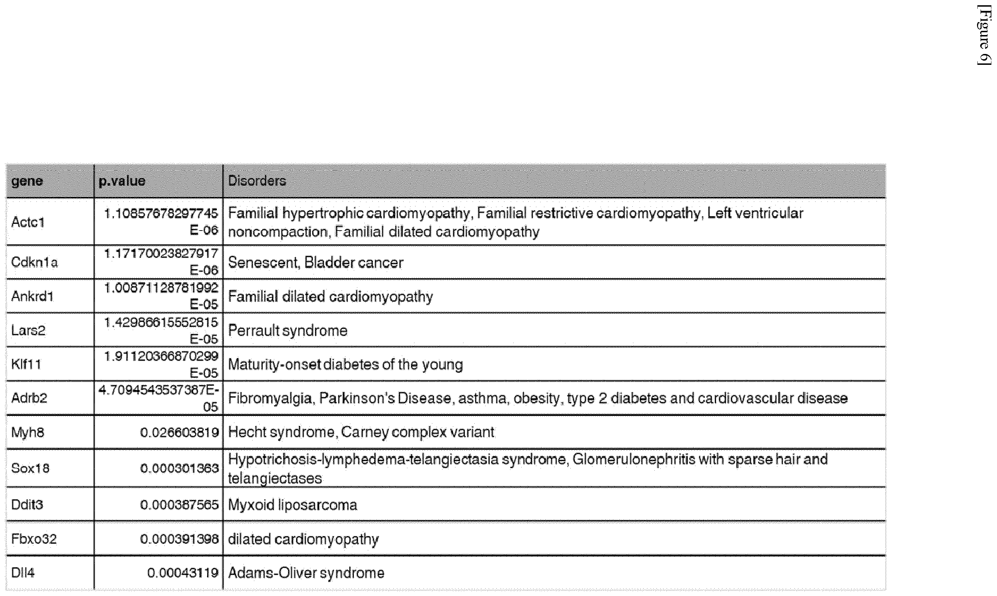

In this example, diseases associated with the top genes showing altered expression in model mice were found by cross-checking with NIH MedlinePlus (Figure 6).

### [Example 2: Ambroxol hydrochloride]

### (Example 2-1: Effects of the compound and effects of combination with exercise therapy)

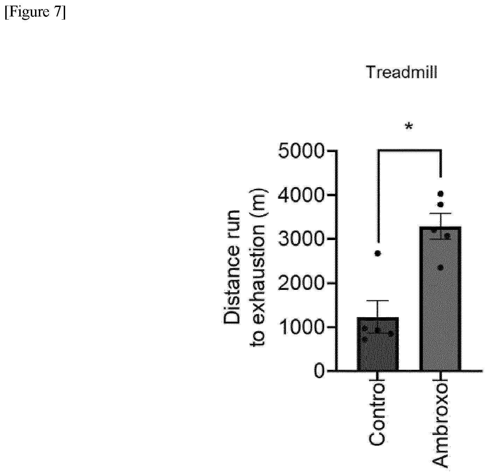

Ambroxol hydrochloride was used in place of L-ethylcysteine hydrochloride, and increase in muscle strength in aged mice was confirmed in the same manner as in Example 1. Ambroxol hydrochloride was orally administered to aged mice from 50 to 77 weeks of age. The dose was 9.21 mg/kg/day. During the administration period, no significant changes in body weight or behavioral abnormalities were observed. After administration of ambroxol hydrochloride at 77 weeks of age, muscle strength (endurance) was measured by the treadmill test; as a result, an increase in muscle strength by means of administration of ambroxol hydrochloride was observed specifically in aged mice (Figure 7).

### (Example 2-2: Improvement in cognitive function)

Cognition and memory of the 77-week-old aged mice of Example 2-2 were evaluated by means of a contextual fear conditional (CFC) test. In the cognition and memory experiment, memory is evaluated in two ways: fear memory and spatial memory; fear memory is evaluated based on the time the subject freezes and stops moving after receiving an electric shock. The animals freeze immediately after electrical stimulation, but on the following day, if the animals are placed in the electric shock machine and do not remember what happened on the previous day, the amount of time the animals freeze decreases (Reference: https://www.ncbi.nlm.nih.gov/books/NBK5223/). Regarding spatial memory, when a mouse is placed on a white disk and a strong light is illuminated thereon, the mouse, which is nocturnal, will try to escape to a dark place. The disk has 20 holes, of which 19 are blocked and only one through which escape is possible. The mouse is then trained for six days to memorize the location of that one hole. The degree to which the mouse could remember the location of the hole was evaluated one day later and one week later (reference: https://www.nature.com/protocolexchange/protocols/349Reiserer).

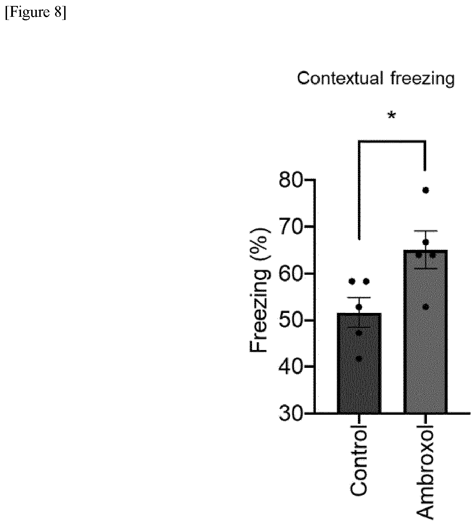

In this example, fear memory was evaluated using the CFC test. Fear memory was assessed by CFC after administration of ambroxol hydrochloride to 77-week-old, aged mice of Example 2-2. The results showed that ambroxol hydrochloride treatment improved CFC Freezing scores and increased cognitive performance specifically in the aged mice (Figure 8).

### (Example 2-3: Alteration in gene expression in muscle by means of administration of ambroxol hydrochloride)

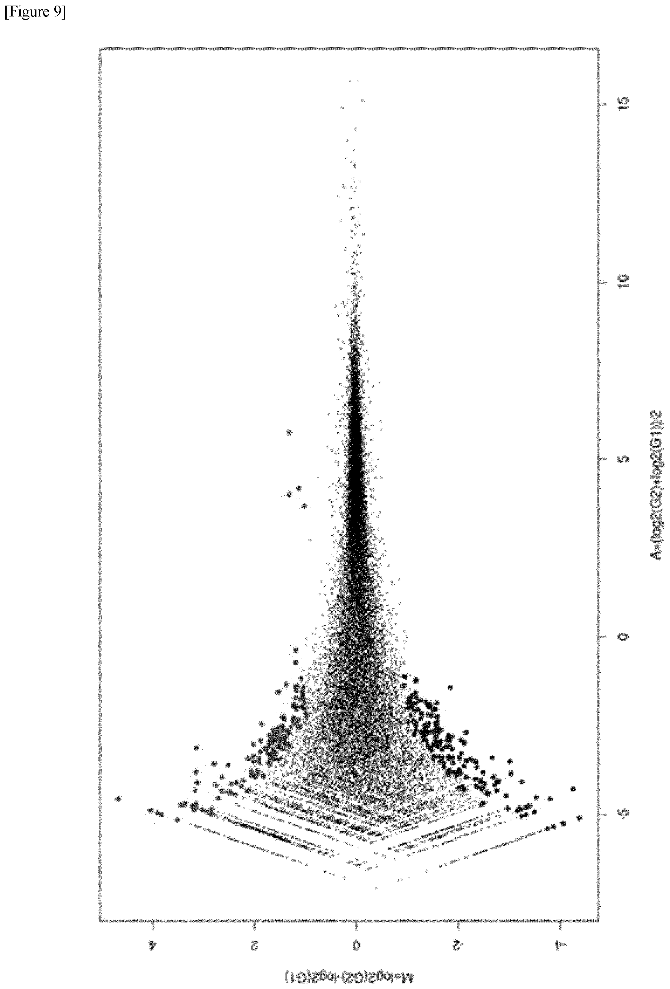

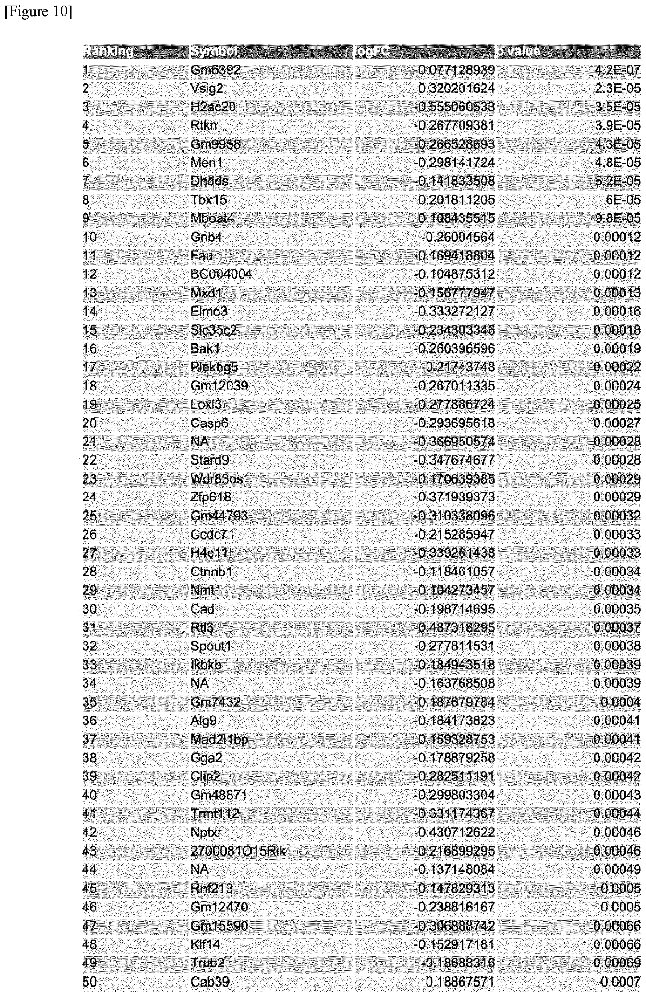

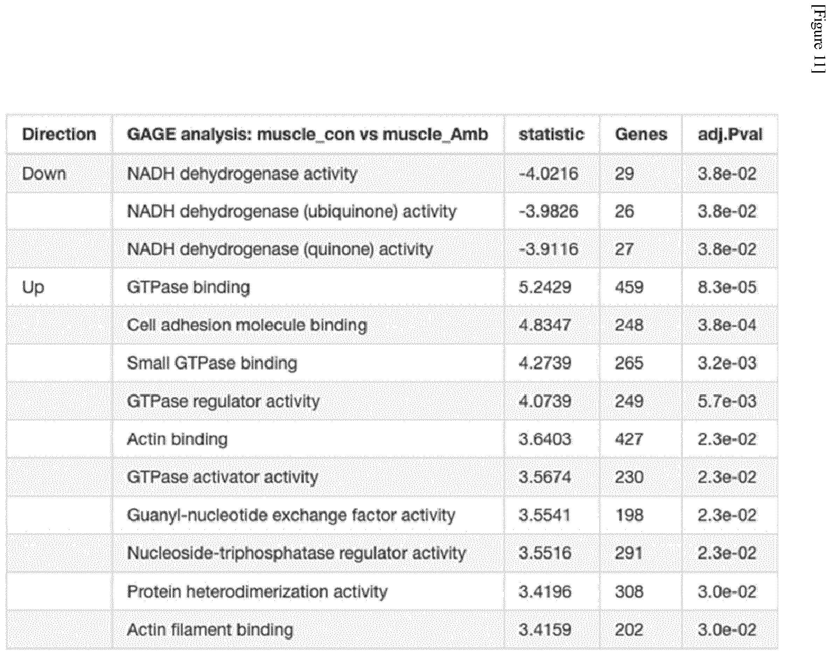

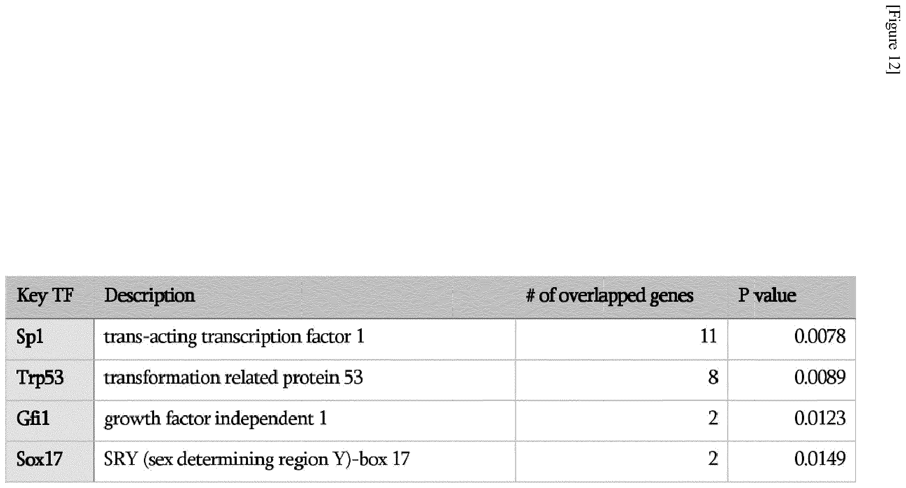

In the present example, gene regulation involved in the ambroxol hydrochloride of the present invention was investigated using model mice to identify genes useful for the diagnosis and treatment of sarcopenia, in the same manner as in Example 1. The results are shown in Figures 9 to 12. Administration of ambroxol hydrochloride increased expression of 164 genes and significantly decreased expression of 204 genes (Figure 9), and changes in gene clusters related to intercellular signals and nerves were observed (Figures 10 and 11). Figure 10 is a diagram showing the top 30 genes with altered gene expression in muscle of mice administered with ambroxol hydrochloride, and Figure 11 shows a pathway analysis diagram of genes with increased gene expression. From the analysis of upstream transcription factors shown in Figure 12, new transcription factors involved in differentiation and cellular senescence were observed as upstream factors. This result suggests the possibility of developing diagnostic and therapeutic agents for sarcopenia using new targets.

### (Example 2-4: Regulation of gene expression in cultured cells)

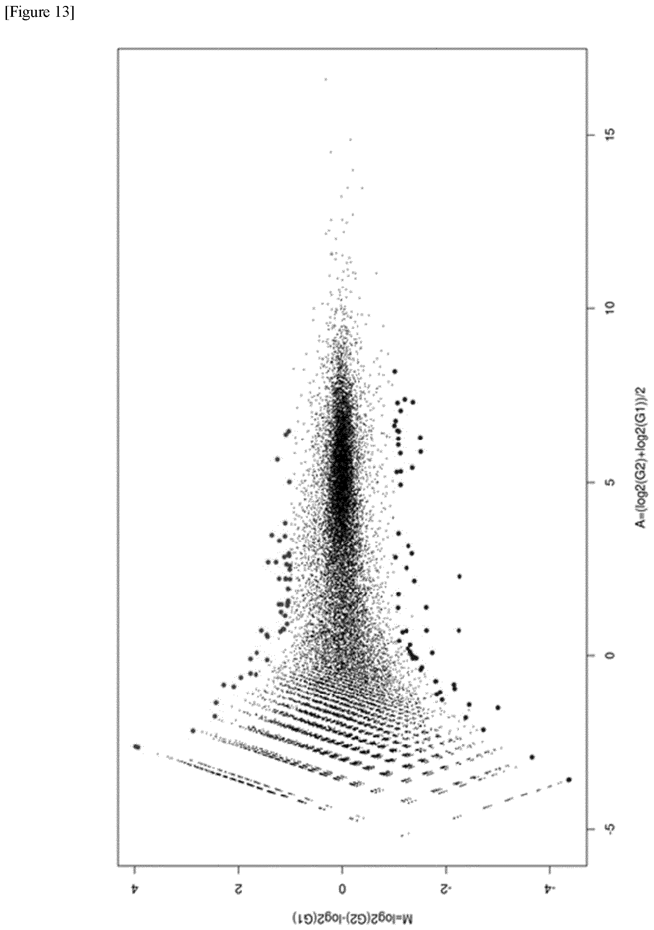

C2C12 cells, which are derived from striated muscle of mice, were cultured and treated with ambroxol hydrochloride. Alterations in gene expression were observed in the same manner as in Example 2-3 by comparing the gene expression levels in cells treated with ambroxol hydrochloride and untreated cells. The results are shown in Figures 13 to 17.

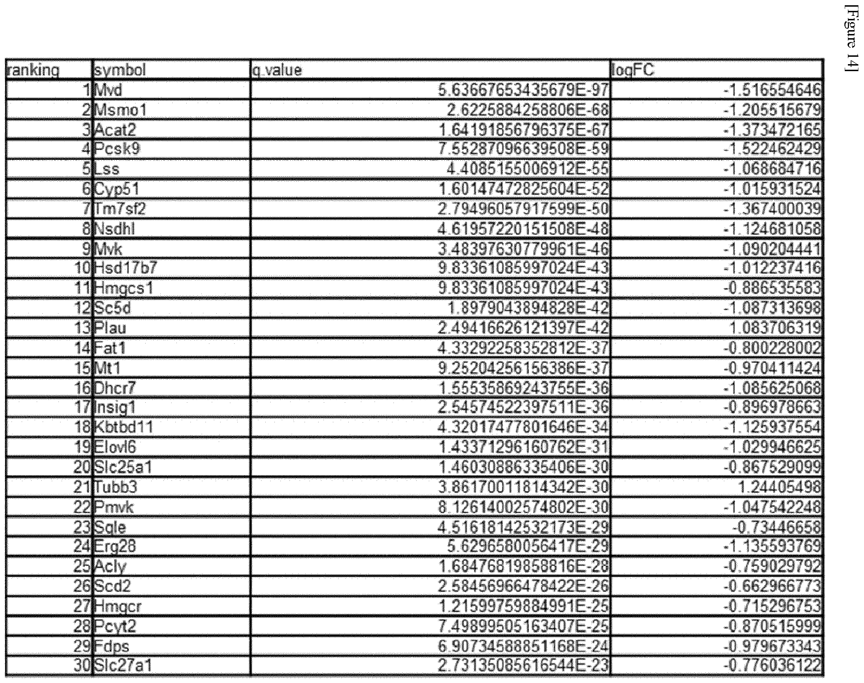

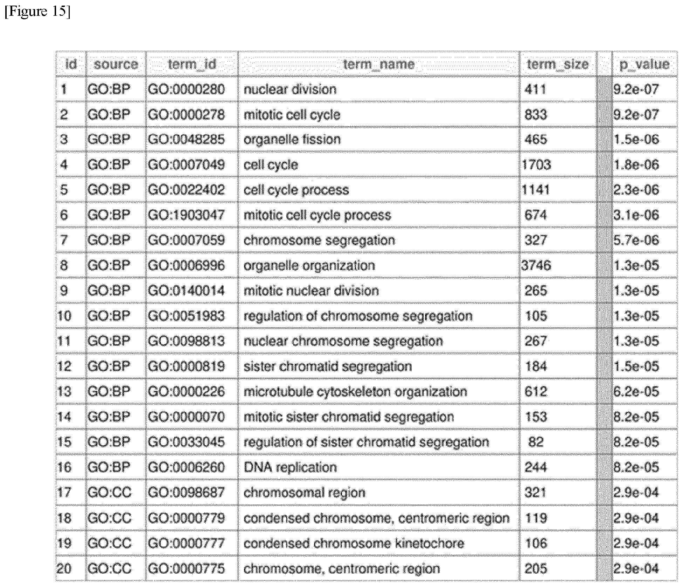

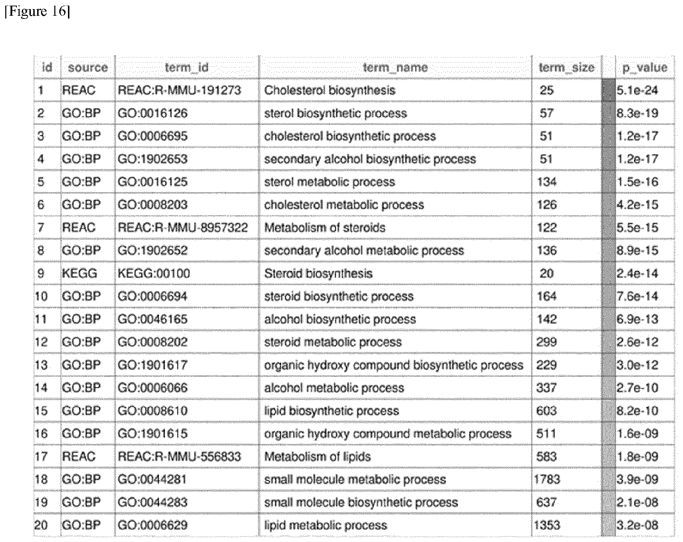

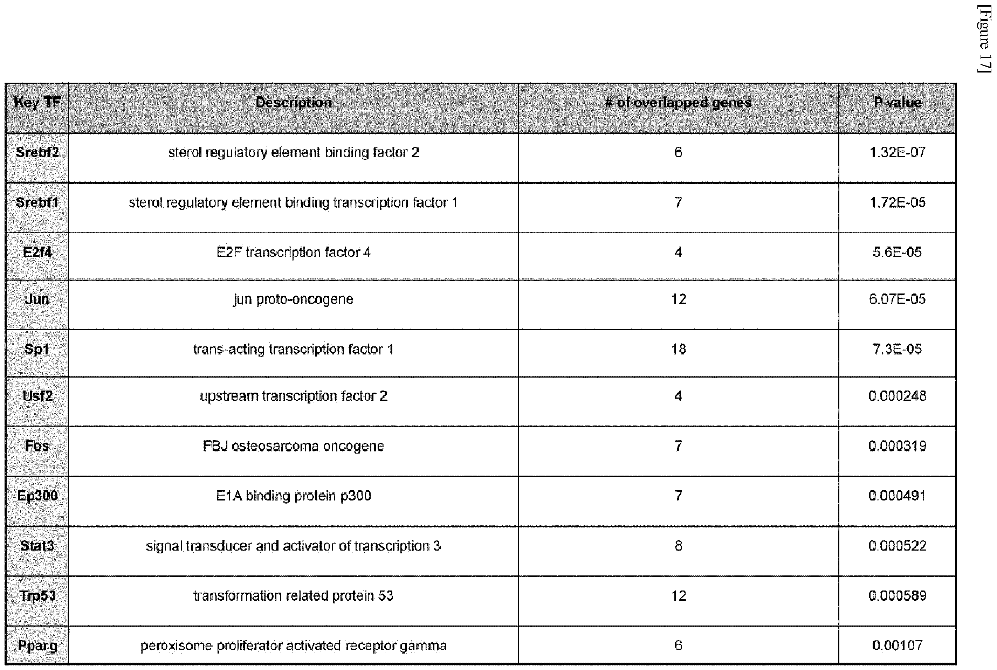

Administration of ambroxol hydrochloride increased expression of 53 genes and significantly decreased expression of 54 genes (Figure 13), and changes in gene clusters related to intercellular signals and nerves were observed (Figures 14 to 17). Figure 14 is a diagram showing the top 30 genes with altered gene expression in muscle of mice administered with ambroxol hydrochloride, and Figure 15 is a diagram showing a pathway analysis diagram of genes with increased gene expression in muscle of mice administered with ambroxol hydrochloride. Figure 16 shows a pathway analysis diagram of genes with decreased gene expression in muscle of mice administered with ambroxol hydrochloride. Figure 17 is a diagram showing gene regulation of muscle in mice administered with ambroxol hydrochloride (with C2C12 cell culture), showing an analysis of upstream transcription factors of genes with altered expression. From the analysis of upstream transcription factors shown in Figure 17, new transcription factors involved in differentiation and cellular senescence were observed as upstream factors. This result suggests the possibility of developing diagnostic and therapeutic agents for sarcopenia using new targets.

### (Example 2-5: Suppression of frailty by means of administration of ambroxol hydrochloride)

The above model mice were orally administered 1000 mg/kg/day of ambroxol hydrochloride from 50 to 81 weeks of age, and frailty associated with aging was confirmed by means of a frailty index test. The evaluated items of the frailty index test are as follows, and an evaluation standard in accordance with clinical symptoms (0 = no symptoms, 0.5 = mild symptoms, 1 = severe symptoms) was employed.
- Hair, skin

| | |
|---|---|
| Alopecia | Whether there is hair loss |
| Loss of fur color | Change in fur color from black to gray, brown, or white |
| Dermatitis | Whether there is skin inflammation |
| Loss of whiskers | Loss of hair and whiskers around nose |
| Coat condition | Condition of coat, smoothness and shine of fur |

- Physical/musculoskeletal

| | |
|---|---|
| Tumors | Presence/absence of tumor |
| Distended abdomen | Abdomen enlarged compared to surrounding |
| Kyphosis | Check by stroking back |
| Tail stiffening | Whether tail wraps freely when stroked, stiffness |
| Gait disorder | Difficulty in walking, wide stance |
| Tremor | Whether there are age-related tremors |
| Forelimb grip strength | Strength of grip on wire mesh when pulled by the tail |
| Body condition score | Check visual signs of bony protuberance, muscle wasting, obesity |

- Vestibulocochlear / auditory

| | |
|---|---|
| Vestibular disturbance | Balance, orientation, acceleration |
| Hearing loss | Whether there is response when clicker is sounded from behind |

- Ocular / nasal

| | |
|---|---|
| Cataracts | Clouding of lens of eye |
| Corneal opacity | Cloudy cornea |
| Eye discharge / swelling | Eyes swollen, abnormal secretions or crusting |
| Microphthalmia | One or both eyes are small and/or sunken |
| Vision loss | Whether the mouse reaches toward the ground when lowered by the tail (also use a ruler) |
| Menace reflex | Whether there is response when foreign object is brought near eyes |
| Nasal discharge | Whether there is nasal discharge |

- Digestive / urogenital

| | |
|---|---|
| Malocclusion | Whether there are malocclusions or overgrown teeth |
| Rectal prolapse | Whether there is protrusion of rectum |
| Vaginal / uterine / penile prolapse | Check genitals |
| Diarrhea | Check cage bedding |

- Respiratory

| | |
|---|---|
| Breathing rate / depth | Check for dyspnea, breath sounds (rales), tachypnea, |

- Discomfort

| | |
|---|---|
| Mouse Grimace Scale | Assess nose bulge, cheek bulge, ear angle, whisker change |
| Piloerection | Whether there is stiff hair or bristling even when stroked |

- Other (compared to reference values)

| | |
|---|---|
| Temperature | Take average of two readings |
| Weight | Measure weight |

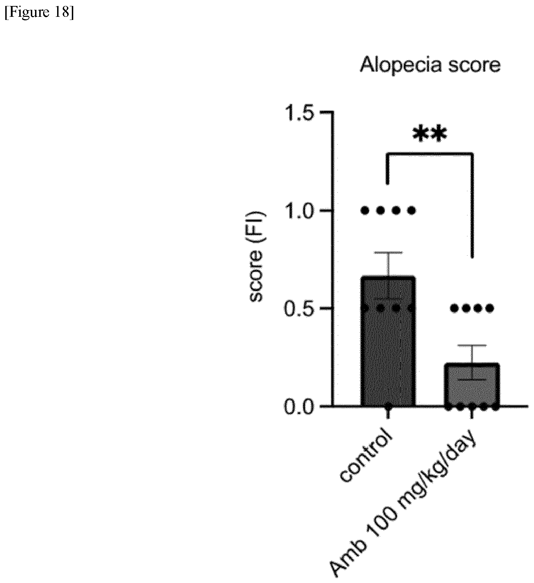

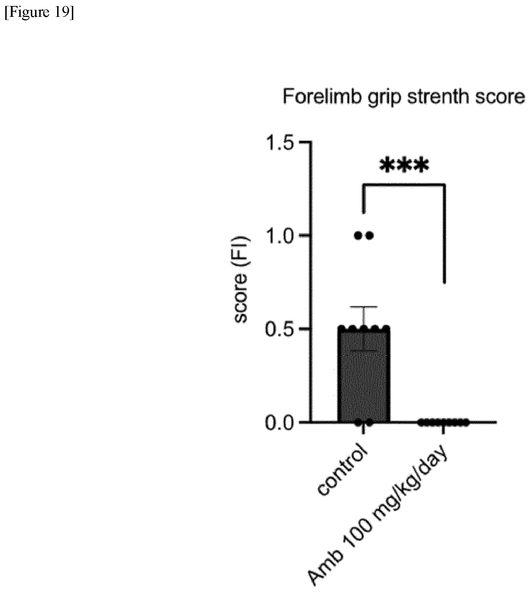

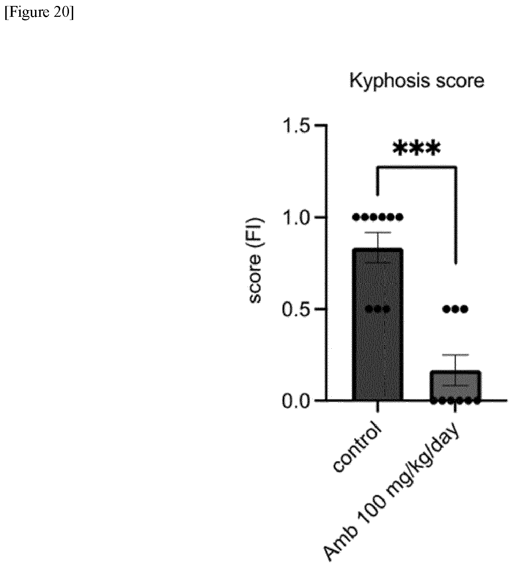

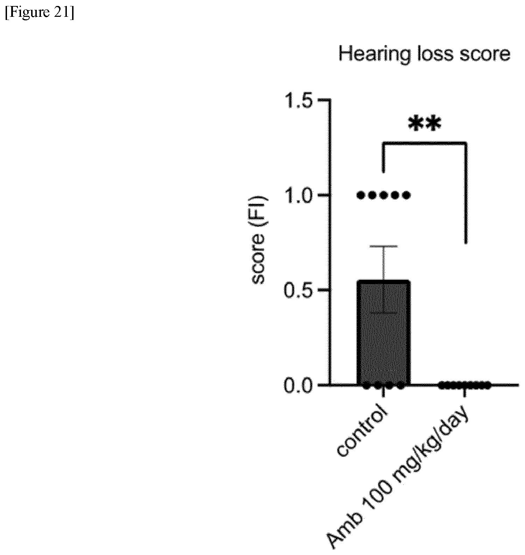

The results are shown in Figures 18 to 21. Figure 18 shows the evaluation results for skin condition (hair and skin), Figure 19 shows muscle strength (grip strength), Figure 20 shows osteoporosis (physical/musculoskeletal), and Figure 21 shows hearing loss. Grip strength was evaluated by means of a measurement of grip strength called the grip test, using a muscle strength measuring device (Muromachi Kikai, MK-380S). Specifically, the mouse was made to grasp a mesh made of wire (wire mesh) with its front and back legs, and its tail was pulled back horizontally little by little until the mouse released the wire mesh. The maximum muscle strength at that time was measured. These results confirmed that administration of ambroxol hydrochloride improved the skin, muscle strength, osteoporosis, coat condition, and the like.

### [Example 3: L-carbocysteine]

### (Example 3-1: Suppression of frailty by L-carbocysteine administration)

The above model mice were given 307.2 mg/kg/day of L-carbocysteine in drinking water for 23 weeks starting at the age of 49 weeks, and frailty index test was performed as in Example 2-5 above to confirm age-related frailty and the like.

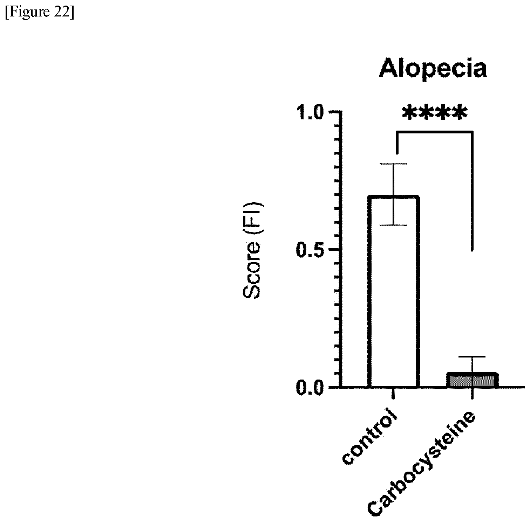

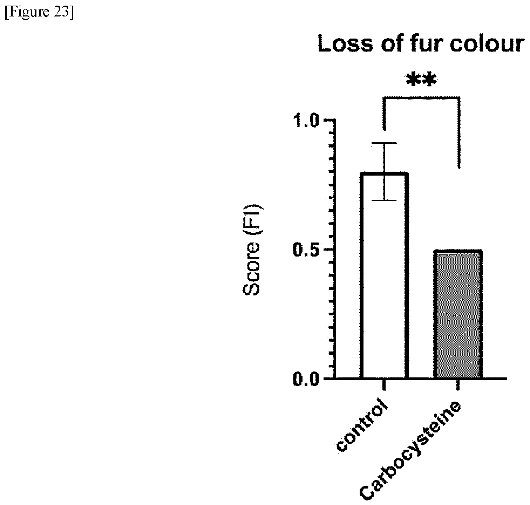

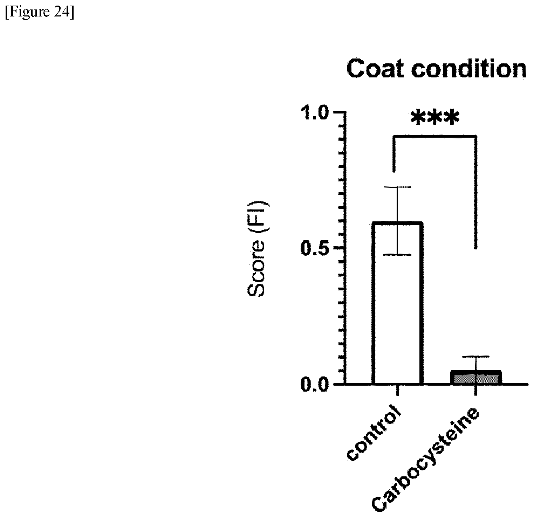

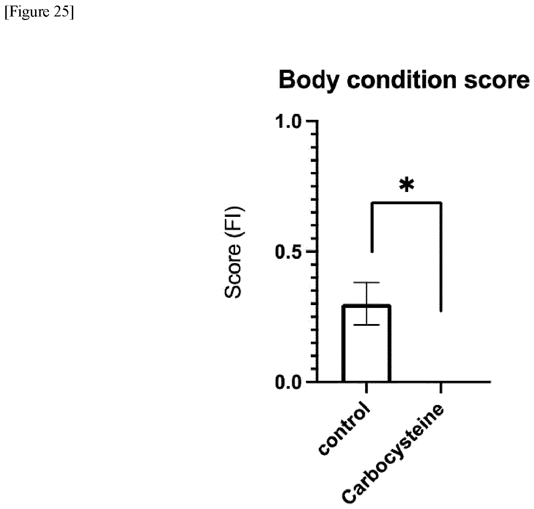

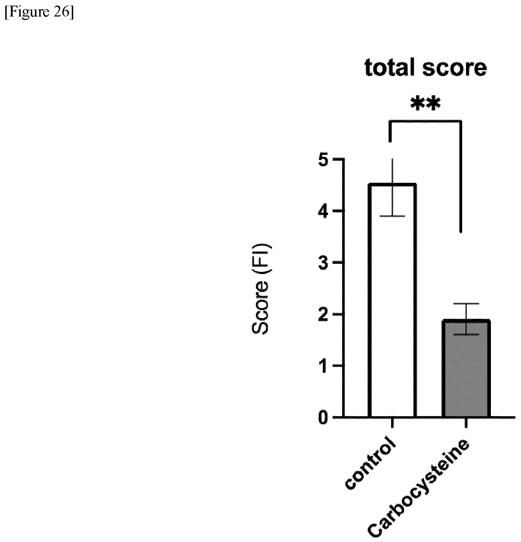

The results of the above frailty index test are shown in Figures 22 through 26. Figure 22 shows the results of alopecia evaluation, Figure 23 shows the results of fur color loss (improvement in gray hair), Figure 24 shows the results of coat condition (improvement in hair loss), Figure 25 shows the results of physical condition score (improvement in bone and muscle), and Figure 26 shows the total score of the frailty index test (improvement in frailty). These results indicate that L-carbocysteine administration improves dermatitis, alopecia, and gray hair, and leads to improvement in physical condition. In addition, since there has been an improvement in the total value, it is expected that frailty will be suppressed.

While the present invention has been illustrated by means of preferred embodiments thereof, it is understood that the scope of the present invention should be construed only by the claims. It is understood that the patents, patent applications, and publications cited in the present Specification are incorporated by reference into this Specification in their entirety as if the contents were specifically set forth herein.

It is clear from the examples described above that the pharmaceutical compositions of the present invention are effective against age-related sarcopenia. More specifically, sarcopenia due to "aging" includes muscle atrophy and decreased muscle function due to inflammation, vascular atrophy, metabolic abnormalities, decreased stem cell function, and imbalance of muscle fiber types, which can be restored by the pharmaceutical compositions of the present invention. Accordingly, it is speculated that the pharmaceutical compositions of the present invention are also effective against sarcopenia such as cancer malignant fluid (sarcopenia due to metabolic abnormalities), infectious diseases (sarcopenia due to inflammation), and chronic renal failure (sarcopenia due to metabolic abnormalities).

Furthermore, from the above examples, it can be inferred that the pharmaceutical compositions of the present invention are effective against, for example, osteoporosis including primary and secondary osteoporosis; dermatitis including pruritus, contact dermatitis, seborrheic dermatitis, sebaceous deficiency dermatitis, eczema coinatum, lichen simplex chronicus, erythroderma, psoriasis and hives; and hearing disorders including age-related hearing loss; white hair; and hair loss, etc.

### Industrial applicability

The present invention can improve sarcopenia-related diseases, disorders, or symptoms, and is expected to be suitably utilized and applied in related industries. The present invention relates to a novel treatment and prevention of sarcopenia-related diseases, disorders, symptoms, etc., and provides compounds, compositions, pharmaceuticals, and treatment methods that are useful and suitable for the novel treatment and prevention of sarcopenia-related diseases, disorders, symptoms, etc. Therefore, the compounds, compositions, and pharmaceuticals according to the present invention can be used (concomitantly used) for purposes such as promoting exercise and rehabilitation, can be widely applied not only to humans but also to animals, and can be incorporated into foods and feeds, for example.

## Claims

1. A pharmaceutical composition for regulating, delaying the progression of, preventing or treating an aging-related abnormality comprising sarcopenia, the composition comprising an expectorant or pharmaceutically acceptable salt thereof or solvate thereof.

2. The pharmaceutical composition according to claim 1, wherein the expectorant comprises one or more compounds selected from the group consisting of L-ethylcysteine, ambroxol, L-carbocysteine, and pharmaceutically acceptable salts thereof.

3. The pharmaceutical composition according to claim 2, wherein the compound is L-ethylcysteine hydrochloride represented by the following general formula (1) and/or ambroxol hydrochloride represented by the following general formula (2):

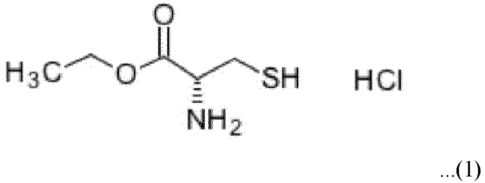

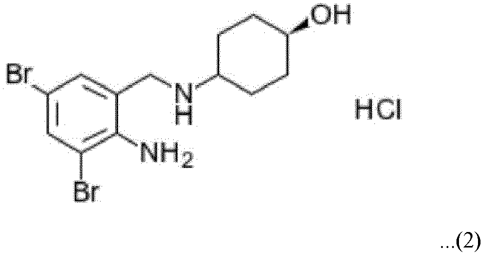

4. The pharmaceutical composition according to claim 2 or 3, wherein the aging-related abnormality comprising sarcopenia comprises at least one selected from the group consisting of a disease, disorder, or symptom caused by impaired muscle strength; a disease, disorder, or symptom caused by impaired energy metabolism; a disease, disorder, or symptom caused by impaired nervous system; and a disease, disorder, or symptom caused by an impaired secretory organ.

5. The pharmaceutical composition according to claim 2 or 3, wherein the aging-related abnormality comprising sarcopenia comprises at least one selected from the group consisting of primary sarcopenia, secondary sarcopenia, and a disease, disorder, or symptom associated therewith.

6. The pharmaceutical composition according to claim 2 or 3, wherein the aging-related abnormality comprising sarcopenia is selected from the group consisting of sarcopenia-related lifestyle diseases (non-wasting diseases), wasting diseases, tumor-related diseases (comprising cancer cachexia), infectious diseases and related diseases such as COVID-19, locomotor diseases, wasting diseases, tumor-related diseases, locomotor diseases, neurodegenerative diseases, cognitive impairments, and aging-related impairments (comprising malnutrition, frailty, spinal and lung injury, inactivity, reduced activity, disuse syndrome, and invasion such as trauma and surgery).

7. The pharmaceutical composition according to claim 2 or 3, wherein the aging-related abnormality comprising sarcopenia is that of a system selected from the group consisting of digestive system (such as digestive tract), circulatory system, respiratory system (such as vocal organs), urinary system, reproductive system, endocrine system, sensory system, cerebral nervous system, and motor system (such as bones, joints, ligaments, and muscles).

8. The pharmaceutical composition according to claim 2 or 3, wherein the aging-related abnormality comprising sarcopenia comprises at least one selected from the group consisting of hair loss and graying hair.

9. The pharmaceutical composition according to claim 2 or 3, wherein the compound or pharmaceutically acceptable salt thereof, or solvate thereof, can be orally administered.

10. The pharmaceutical composition according to claim 2 or 3, wherein the regulating, delaying of progression, preventing, or treating comprises regulation, delay of progression, prevention, or treatment of at least one of muscle mass loss, decreased (muscle) endurance, dementia, bone density loss, osteoporosis, and hair loss.

11. The pharmaceutical composition according to claim 2 or 3, wherein the regulating, delaying of progression, preventing, or treating comprises regulation, delay of progression, prevention, or treatment of at least one of muscle mass loss and decreased (muscle) endurance.
